# EUROPÄISCHE PATENTSCHRIFT

(11) **EP 3 545 468 B1**
(45) Veröffentlichungstag und Bekanntmachung des Hinweises auf die Patenterteilung: **11.06.2025**
(21) Anmeldenummer: 17822539.7
(22) Anmeldetag: 23.11.2017
(51) Int. Cl.: G06V 10/44, G06F 18/2137

(54) **VERFAHREN UND SYSTEM ZUM ERSTELLEN EINER MEDIZINISCHEN BILDDATENBANK MITTELS EINES KONVOLUTIONELLEN NEURONALEN NETZWERK**
METHOD AND SYSTEM TO BUILD A MEDICAL IMAGES DATABASE USING A CONVOLUTIONAL NEURAL NETWORK
MÉTHODE ET SYSTÈME POUR CONSTRUIRE UNE BASE DE DONNÉES D'IMAGES MÉDICALES EN UTILISANT UN RÉSEAU NEURONAL CONVOLUTIONNEL

(30) Priorität: 25.11.2016 AT 510722016
(43) Veröffentlichungstag der Anmeldung: 02.10.2019
(73) Patentinhaber: contextflow GmbH, 1050 Vienna (AT)
(72) Erfinder: DONNER, Rene, 1200 Wien (AT)
(74) Vertreter: J A Kemp LLP
(86) Internationale Anmeldenummer: PCT/AT2017/060312
(87) Internationale Veröffentlichungsnummer: WO 2018/094438

(56) Entgegenhaltungen:
- US-A1- 2004 165 767
- US-B1- 6 760 714
- XUFENG HAN ET AL: "MatchNet: Unifying feature and metric learning for patch-based matching", 2015 IEEE CONFERENCE ON COMPUTER VISION AND PATTERN RECOGNITION (CVPR), IEEE, 7 June 2015 (2015-06-07), pages 3279 - 3286, XP032793776, DOI: 10.1109/CVPR.2015.7298948
- PRASOON ADHISH ET AL: "Deep Feature Learning for Knee Cartilage Segmentation Using a Triplanar Convolutional Neural Network", ECCV 2016 CONFERENCE; [LECTURE NOTES IN COMPUTER SCIENCE; LECT.NOTES COMPUTER], SPRINGER INTERNATIONAL PUBLISHING, CHAM, vol. 8150 Chap.31, no. 558, 22 September 2013 (2013-09-22), pages 246 - 253, XP047042067, ISSN: 0302-9743, ISBN: 978-3-642-33485-6
- LIAO SHU ET AL: "Representation Learning: A Unified Deep Learning Framework for Automatic Prostate MR Segmentation", ECCV 2016 CONFERENCE; [LECTURE NOTES IN COMPUTER SCIENCE; LECT.NOTES COMPUTER], SPRINGER INTERNATIONAL PUBLISHING, CHAM, vol. 8150 Chap.32, no. 558, 22 September 2013 (2013-09-22), pages 254 - 261, XP047042069, ISSN: 0302-9743, ISBN: 978-3-642-33485-6

## Beschreibung

Die Erfindung betrifft ein Verfahren und ein System zur Erstellung einer medizinischen Bilddatenbank gemäß dem Oberbegriff des Patentanspruchs 1 bzw. des Patentanspruchs 13.

Die Erfindung betrifft das technische Gebiet der, insbesondere medizinischen, Bildverarbeitung, beispielsweise von medizinischen Bilddaten, die aus Methoden der bildgebenden Diagnostik resultieren. Dabei handelt es sich beispielsweise um zwei- oder dreidimensionale CT- oder MRT-Aufnahmen, Ultraschallaufnahmen oder mikroskopische Aufnahmen. Ebenso betreffen bevorzugte Aspekte der Erfindung Verfahren zur Verarbeitung medizinischer Texte wie beispielsweise von Fachzeitschriften sowie von schriftlichen Diagnose-Texten, Patienten berichten.

Die wesentlichen Herausforderungen auf diesem Gebiet bestehen in der Identifikation der für die Diagnose relevanten Merkmale, mit dem eine zuverlässige Unterstützung des betreffenden Arztes möglich ist.

Besonders im medizinischen Bereich ist die Suche nach Bilddaten, wie beispielsweise Röntgenaufnahmen oder computertomographischen Aufnahmen, welche ähnliche anatomische Strukturen, Veränderungen oder Krankheitssymptome darstellen und somit ähnlichen Krankheitsfällen zuzuordnen sind, sehr schwierig und zeitaufwändig, insbesondere, wenn nur visuelle Informationen als Grundlage für die Suche zur Verfügung stehen, da von einer Krankheit oft nur kleinräumige Veränderungen im menschlichen Körper hervorgerufen werden.

Besonders im klinischen Bereich ist jedoch eine präzise, auch in umfangreichen medizinischen Datenbanken schnell durchführbare Suche nach ähnlichen Bilddaten wünschenswert, um möglichst rasch für einen aktuellen Krankheitsfall ähnliche Krankheitsfälle ermitteln und darauf aufbauend eine Diagnose für den aktuellen Krankheitsfall stellen zu können.

US 6,760,714 B1 beschreibt ein Verfahren in dem Bildmerkmale durch Durchführen von Wavelet-Transformationen an Abtastpunkten von in elektronischer Form gespeicherten Bildern erzeugt werden. Mehrere Wavelet-Transformationen an einem Punkt werden kombiniert, um einen Bildmerkmalsvektor zu bilden. Ein prototypischer Satz von Merkmalsvektoren oder Atomen wird aus dem Satz von Merkmalsvektoren abgeleitet, um ein "Atomvokabular" zu bilden. Die prototypischen Merkmalsvektoren werden unter Verwendung eines Vektorquantisierungsverfahrens abgeleitet, in dem auch ein Vektorquantisierungsnetzwerk erzeugt wird. Das atomare Vokabular wird verwendet, um neue Bilder zu definieren. Die Bedeutung wird zwischen Atomen im atomaren Vokabular hergestellt. Jedem Atom sind hochdimensionale Kontextvektoren zugeordnet. Die Kontextvektoren werden dann als Funktion der Nähe und des gemeinsamen Auftretens jedes Atoms zu anderen Atomen im Bild trainiert. Nach dem Training werden die Kontextvektoren, die den Atomen zugeordnet sind, die ein Bild bilden, kombiniert, um einen Zusammenfassungsvektor für das Bild zu bilden. Bilder werden unter Verwendung einer Anzahl von Abfragemethoden abgerufen (z. B. Bilder, Bildteile, Vokabularatome, Indexausdrücke).

Aufgabe der Erfindung ist daher, ein Verfahren zur effizienten Erstellung von medizinischen Bilddatenbanken zur Verfügung zu stellen, sodass diese präzise und rasch durchsuchbar sind. Insbesondere ist es Aufgabe der Erfindung, eine auf inhaltlichen Kriterien basierende Bildsuche vorzunehmen. Von Vorteil ist es in diesem Zusammenhang, wenn hierfür auch auf moderne Methoden des deep-learning, wie beispielsweise convolutional neural networks (CNN) oder auf recurrent neural networks (RNN) zurückgegriffen werden kann.

Die Erfindung löst diese Aufgabe durch ein Verfahren zum Erstellen einer medizinischen Bilddatenbank, wobei a) Datensätze vorgegeben werden, die Teilbilder von zwei- oder mehrdimensionalen Ausgangsbildern von Teilen des menschlichen Körpers umfassen, und wobei
- für jedes Teilbild eines Ausgangsbilds die jeweilige Position in einem anatomischen Referenzatlas bekannt ist, und
- die einzelnen Ausgangsbilder oder Teilbilder gegebenenfalls mit textuellen, numerischen oder semantischen Zusatzinformationen versehen sind, und
- ein Teilbild auch einem gesamten Ausgangsbild entsprechen kann, mit den kennzeichnenden Merkmalen des Patentanspruchs 1.

Erfindungsgemäß ist dabei vorgesehen, dass
b) eine Projektion zur Gewinnung von Feature-Vektoren aus den Teilbildern erstellt wird,
   - die, insbesondere visuell oder semantisch, ähnliche Teilbilder auf Feature-Vektoren mit geringem Abstand abbildet, und
   - wobei zur Vorbereitung der Ausführung der Projektion ein neuronales Netzwerk, insbesondere ein Convolutional-Neural-Network, auf der Basis vorgegebener Lern-Teilbilder erstellt wird, wobei die Datensätze oder ein Teil der Datensätze im Rahmen eines Metric-Learning-Verfahrens vom neuronalen Netzwerk dazu genutzt werden, die Projektion und das Erstellen der Feature-Vektoren aus Lern-Teilbildern oder Gruppen von Lern-Teilbildern sowie einer zu erreichenden vorgegebenen Ähnlichkeit zwischen den Lern-Teilbildern zu erlernen, wobei dem betreffenden Metric-Learning-Verfahren [schroff2015] eine oder mehrere der folgenden Vorgaben zugrunde gelegt werden:
   - Vorgabe von n-Tupeln von Lern-Teilbildern oder Gruppen von Lern-Teilbildern als ähnlich, die zueinander geringfügig verschoben, rotiert, verzerrt oder gestreckt sind und ausgehend vom selben Ausgangsbild erstellt wurden und/oder
   - Vorgabe von n-Tupeln von Lern-Teilbildern oder Gruppen von Lern-Teilbildern als ähnlich, die ausgehend vom selben Teilbereich des Ausgangsbilds erstellt werden, wobei zumindest eines der Lern-Teilbilder gegenüber dem Teilbereich des Ausgangsbilds derart modifiziert wird, dass die Lern-Teilbilder unterschiedliches Rauschen und/oder, unterschiedliche Bildintensität und/oder unterschiedlichen Kontrast aufweisen und/oder
   - Vorgabe von n-Tupeln von aus demselben Ausgangsbild oder Gruppen von Ausgangsbildern stammenden Teilbereichen als Lern-Teilbilder, wobei die zu erreichende Ähnlichkeit zwischen den jeweiligen Lern-Teilbildern Bildern des n-Tupels vom räumlichen Abstand der betreffenden Teilbereiche im Ausgangsbild abhängt, wobei insbesondere Lern-Teilbilder umso ähnlicher angesehen werden, je näher die betreffenden Teilbereiche im Ausgangsbild beieinander liegen, und/oder
   - Erstellen einer komprimierten Repräsentation der in einem Teilbild enthaltenen Informationen
   - Lern-Teilbildern oder Gruppen von Lern-Teilbildern vom selben Ausgangsbild 4a, 4b, 4c oder von unterschiedlichen Ausgangsbildern 4a, 4b, 4c, die aufgrund von mit den jeweiligen Ausgangsbildern 4a, 4b, 4c hinterlegten externen Merkmalen wie textuellen, numerischen oder semantischen Informationen als ähnlich anzusehen sind.
c) wobei auf die Teilbilder der Datensätze und/oder auf eine Anzahl weiterer Teilbilder weiterer Datensätze die Projektion angewendet wird und dementsprechend für diese Teilbilder jeweils zumindest ein Feature-Vektor erhalten wird, und
d) wobei die derart erstellten Feature-Vektoren, insbesondere verknüpft mit den Ausgangsbildern und/oder Datensätzen oder weiteren Datensätzen, in einer Indexdatenstruktur hinterlegt werden,
wobei die jeweilige Position der Teilbilder eines Ausgangsbilds bezogen auf den menschlichen Körper ermittelt wird, und insbesondere, wobei die Informationen zur Position der Teilbilder von einem neuronalen Netzwerk dazu genutzt werden, eine Projektion zur Schätzung der Positionen von Teilbildern zu erlernen,
- wobei die Projektion gelernt wird mit der Zielfunktion, dass durch Abbildung von Paaren oder Gruppen von Teilbildern die räumliche Konstellation der Paare/Gruppen vor und nach der Projektion ähnlich ist und
- wobei die Projektion basierend auf einer bekannten Abbildung von Teilbildern auf Positionen gelernt wird
- wobei zusätzlich zu den Feature-Vektoren die gelernte oder bekannte Positionsinformation in der Datenbank abgespeichert wird
- wobei bei Vorliegen einer Suchanfrage die gesuchte Position im Körper ermittelt wird und in der Datenbank nach Feature-Vektoren von Teilbildern gesucht wird, für die dieselbe Position in deren Datensätzen hinterlegt ist oder deren Position einen vom Benutzer vorgegebenen Abstandsschwellenwert zur gesuchten Position nicht überschreitet.

Von besonderem Vorteil ist die Erfindung, weil es - beispielsweise für einen Radiologen - mit so erstellten der Bilddatenbank möglich ist, für ein vorgegebenes medizinisches Bild oder Teilbild ähnliche in der erstellten Datenbank abgespeicherte Teilbilder aufzufinden, die gegebenenfalls mit zusätzlichen Informationen versehen sind. Ebenso können der Datenbank auch ähnliche Fälle von Teil-Bildern entnommen werden, die - beispielsweise für den Radiologen - eine verbesserte Informationen enthalten. Durch die Erfindung ist eine besonders rasche und effektive Suche in der Datenbank nach Bilddaten von Krankheitsfällen betreffend bestimmte Körperteile ermöglicht.

Um beispielsweise auch Informationen aus der Krankenakte eines Patienten in der Datenbank effizient zu hinterlegen, kann vorgesehen sein, dass in den Datensätzen Zusatzinformationen hinterlegt werden, wobei die Zusatzinformationen als Text-Informationen und/oder semantische Informationen und/oder numerische Informationen vorgegeben werden und
- dass optional bei Vorliegen von Text-Informationen oder numerischen Informationen die Text-Informationen oder numerischen Informationen als Tags und/oder semantische Repräsentationen in der Datenbank hinterlegt werden

Um Feature-Vektoren welche besonders präzise und rasch durchsuchbar sind, aus den Teilbildern gewinnen zu können, kann vorgesehen sein, dass die Zusatzinformationen vom neuronalen Netzwerk zur Erstellung der Projektion genutzt werden, wobei die Projektion derart erstellt wird, dass Lern-Teilbilder als ähnlich vorgegeben werden, die von Ausgangsbildern stammen oder Teilbildern entsprechen, denen dieselbe Zusatzinformation zugeordnet ist.

Zur Durchführung einer präzisen, rechenleistungseffizienten Suche in der Datenbank beispielsweise nach Bilddaten zu bestimmten Krankheitsfällen kann vorgesehen sein, dass zur Erstellung einer Suchanfrage vom Benutzer Text-Informationen, numerische Informationen und/oder semantische Informationen angegeben werden,
- dass optional die Text-Informationen oder numerischen Informationen in Tags und/oder semantische Repräsentationen umgewandelt werden
- dass bei Vorliegen einer Suchanfrage in der Datenbank nach Feature-Vektoren gesucht wird, die ähnliche Tags und/oder semantische Repräsentationen besitzen und
- dass zur Erstellung einer Suchanfrage zumindest ein Abfragebild, insbesondere eines interessierenden Bereichs, aus zumindest einem zwei- oder mehrdimensionalen Untersuchungsbild oder in einer Untersuchungsbildsequenz ausgewählt wird,
- für das Abfragebild nach Schritt c des Anspruchs 1 ein Feature-Vektor nach der erlernten Projektion ermittelt wird,
- in der Datenbank nach Datensätzen mit Feature-Vektoren gesucht wird, die unter Zugrundelegung einer vorgegebenen Metrik in der Nähe des Feature-Vektors des Abfragebilds liegen, und

als Ergebnis der Suchanfrage Datensätze ausgegeben werden, deren Teilbilder ein ähnliches Aussehen wie der Auswahlbereich aufweisen oder die semantisch relevant sind.

Um auch ausgehend von beispielsweise dreidimensionalen Bilddaten eine Suche in einer Datenbank umfassend zweidimensionale Bilddaten durchführen zu können, kann vorgesehen sein, dass zur Suche ähnlicher Bilder für ein Untersuchungsbild oder eine Untersuchungsbildsequenz durch Erstellung eines Schnitts ein dimensionsreduziertes und gegebenenfalls in seinen Abmessungen reduziertes Abfrageteilbild erstellt wird und dieses Abfrageteilbild für die Suchanfrage verwendet wird.

Um die Anzahl an Suchergebnissen möglichst gering halten zu können, sodass nur die für den Benutzer relevantesten Suchergebnisse ermittelt und ausgegeben werden, kann vorgesehen sein, dass bei der Suche nach ähnlichen Feature-Vektoren in der Datenbank Suchergebnisse aufgrund der in den zugehörigen Datensätzen in der Datenbank hinterlegten Tags und/oder semantischen Repräsentationen ausgeschlossen werden, insbesondere, dass Suchergebnisse unter vom Benutzer für die Tags und/oder semantischen Repräsentationen vorgegeben Kriterien ausgeschlossen werden und/oder dass als Ergebnis der Suchanfrage eine Rangfolge der jeweils ermittelten Datensätze nach deren Ähnlichkeit oder Übereinstimmung zu den Tags und/oder semantischen Repräsentationen der Suchanfrage und/oder nach deren, und/oder durch den Abstand der betreffenden Feature-Vektoren bestimmten, Ähnlichkeit zu zumindest einen Abfragebild der Suchanfrage erstellt und ausgegeben wird.

Damit einem Benutzer nicht nur Bilddaten, sondern auch zusätzliche Informationen oder statistische Daten als Ergebnis der Suchanfrage zur Verfügung gestellt werden, kann vorgesehen sein, dass als Ergebnis der Suchanfrage die in den jeweiligen Datensätzen als Tags hinterlegten Text-Informationen, numerischen Informationen und/oder die als semantische Repräsentationen hinterlegten semantischen Informationen ausgegeben werden und/oder
- dass die Tags der zum Auswahlbereich ähnlichen Teilbilder statistisch ausgewertet werden und das statistische Ergebnis ausgegeben wird.

Zur Gewährleistung des Schutzes von Patientendaten, wenn beispielsweise von einem Benutzer eine Suchanfrage per Internet an die Datenbank übermittelt wird, kann vorgesehen sein, dass die der Suchanfrage zugrunde liegenden Untersuchungsbilder und/oder Untersuchungsbildsequenzen und/oder Text-Informationen und/oder numerischen Informationen und/oder semantischen Informationen vor der Übermittlung der Suchanfrage durch den Benutzer an die Datenbank anonymisiert werden.

Um die zu einer Suchanfrage ermittelten Ergebnisse für den Benutzer strukturiert aufzubereiten, kann vorgesehen sein, dass ohne eine Suchanfrage direkt aufgrund der in der Datenbank vorhandenen Daten in der Datenbank Gruppen von Teilbildern und/oder Text-Informationen und/oder numerischen Informationen und/oder semantischen Informationen mit ähnlichen Feature-Vektoren erstellt und ausgegeben und gegebenenfalls Zusatzinformationen hinsichtlich dieser Gruppen ausgegeben werden, und/oder
- dass auf die Suchanfrage eines Benutzers in der Datenbank benachbarte Gruppen von Teilbildern und/oder Text-Informationen und/oder numerischen Informationen und/oder semantischen Informationen mit ähnlichen Feature-Vektoren ermittelt und ausgegeben werden und gegebenenfalls Zusatzinformationen hinsichtlich dieser Gruppen ausgegeben werden.

Um eine kaskadenartige Suche aufeinanderfolgend in mehreren Datenbanken durchführen zu können, wobei jede weitere Suchanfrage jeweils auf den bereits ermittelten Suchergebnissen aufbaut, kann vorgesehen sein, dass die als Ergebnis einer ersten Suchanfrage an die Datenbank ermittelten Suchergebnisse, insbesondere Teilbilder, Gruppeninformationen, Textinformationen, numerische Informationen und/oder semantische Informationen, zur Erstellung zumindest einer weiteren Suchanfrage verwendet werden, und
- dass die weitere Suchanfrage an zumindest eine weitere Datenbank übermittelt wird, und/oder
- dass zur Erstellung der zumindest einen weiteren Suchanfrage an die zumindest eine weitere Datenbank weitere als Ergebnis der ersten Suchanfrage an die Datenbank ausgegebene Informationen verwendet werden,
- insbesondere, dass ausgegebene statistische Ergebnisse und/oder Ausgangsbilder und/oder ähnliche Teilbilder verwendet werden, wobei die Dimension der ausgegebenen Ausgangsbilder und/oder der ausgegebenen Teilbilder für die weitere Suchanfrage in der zumindest einen weiteren Datenbank gegebenenfalls reduziert wird.

Zur besonderes präzisen Erstellung einer Suchanfrage unter Einbeziehung von Suchergebnissen einer Datenbank, auf die der Benutzer keinen direkten Zugriff hat, kann vorgesehen sein, dass einzelne Datensätze der Datenbank ausschließlich für die Bildung einer Projektionsfunktion herangezogen werden, dem Benutzer jedoch nicht als Resultate von Abfragen zur Ansicht zur Verfügung gestellt werden.

Um auch Ausgangs- oder Teilbilder deren Pixel- oder Voxelabmessungen unbekannt sind, zur Erstellung der Datenbank bzw. zur Erstellung einer Suchanfrage an die Datenbank nutzen zu können, kann vorgesehen sein, dass in den Datensätzen Größenangaben betreffend die Pixelabmessungen oder Voxelabmessungen der Ausgangsbilder oder Teilbilder hinterlegt werden, oder
dass für die Ausgangsbilder oder Teilbilder die Pixelabmessungen oder Voxelabmessungen vorgegeben werden, indem nach ähnlichen, insbesondere vom selben Körperteil herrührenden, Referenz-Ausgangs- oder Referenz-Teilbildern mit bekannten Pixel- oder Voxelabmessungen gesucht wird
- anschließend durch Bildvergleich eine Skalierung gesucht wird, mit der das Ausgangs- oder Teilbild mit dem Referenz-Ausgangs- oder Referenz-Teilbild optimal in Übereinstimmung gebracht werden kann und
- ausgehend von dieser Skalierung und den bekannten Pixelabmessungen oder Voxelabmessungen des Referenz-Ausgangs- oder Referenz-Teilbilds die Pixelabmessungen oder Voxelabmessungen des Ausgangsbilds oder Teilbilds ermittelt und in der Datenbank abgespeichert werden.

In einer bevorzugten Ausführungsform der Erfindung können Bilder aufgefunden werden, die zu einem vorgegebenen Suchbild ähnlich sind.

In einer weiteren vorteilhaften Ausführungsform der Erfindung können Bilder, die für eine Abfrage relevant sind basierend auf einer durch Training erlernten Zielähnlichkeit erlernt werden, diese Zielähnlichkeit kann durch Training basierend auf bekannten Bildern erlernt werden. Insbesondere kann auch eine Abstandsfunktion, die einen Abstand zwischen zwei Bildern angibt, durch Training erlernt werden.

Eine weitere vorteilhafte Ausführungsform der Erfindung ermöglicht eine verbesserte Bestimmung einer Abstandsfunktion zwischen zwei Bildern basierend auf einer verbesserten Datengrundlage umfassend Informationen über den betreffenden Patienten, Kurven, Diagnosen, Daten über den Krankheitsverlauf, Prognosen sowie Bilddaten. Dabei kann nach dem Training eine ungefähre Zielähnlichkeit basierend auf einem Teil der Daten ermittelt werden, beispielsweise lediglich auf den biomedizinischen Bilddaten. Diese Zielähnlichkeit kann genutzt werden, um später Diagnosen oder Prognosen über den Krankheitsverlauf zu erstellen. Diese Ausführungsform der Erfindung ist dazu in der Lage, eine Abstandsfunktion zu erstellen, die die wesentliche Charakteristik der Zielähnlichkeit auch basierend auf nur teilweise vorhandenen Daten, wie insbesondere ausschließlich auf den Bilddaten, ermittelt.

In einer bevorzugten Ausführungsform der Erfindung ist es auch möglich, Training basierend auf Ähnlichkeits- oder Distanzfunktionen, die Erstellung bzw den Erhalt eines Index ausschließlich basierend auf CNNs für die Bildverarbeitung und RNNs für die Textverarbeitung zu erstellen.

Ein weiterer Vorteil von bevorzugter Ausführungsformen der Erfindung ist das Auffinden von relevanten Fällen, Patenten oder Patenientendatenbasierend auf zur Verfügung stehenden Bildinformationen eines Ausgangsfalls bzw Abfragefalls, wobei dem Benutzer bei der Abfrage lediglich ein Bild als Suchbasis zur Verfügung steht. Eine direkte Zuordnung zwischen ähnlichen Fällen ist aufgrund semantischer Unterschiede in den Daten üblicherweise nur sehr schwer zu modellieren.

In einer bevorzugten Ausführungsform der Erfindung kann eine Abstandsfunktion zur Reihung von Suchresultaten basierend auf einer vom Benutzer angegebenen Suchregion innerhalb des Bilds, gegebenenfalls in Kombination mit weiteren Patienteninformationen wie beispielsweise Alter oder Geschlecht ermittelt werden.

In einer weiteren Ausführungsform der Erfindung können die für den Benutzer am meisten relevanten bzw höchstgereihten Ergebnisse visuell dargestellt werden. Diese Ergebnisse können vom Benutzer basierend auf seiner Erfahrung bestätigt werden.

Von besonderem Vorteil ist es, dass einzelne Ausführungsformen der Erfindung eine Vielzahl von Zusammenhängen lernen, einen Index erstellen, eine Abstandsfunktion ermitteln und statistische Auswertungen und Modellierungen auf der Grundlage von Daten erlernen können, die im täglichen Betrieb eines Spitals anfallen, ohne dass hierfür manuelle Annotation vorgenommen werden bräuchte.

Von besonderem Vorteil ist es auch, dass in einzelnen Ausführungsformen der Erfindung zusätzliche Datenquellen, wie beispielsweise Fachartikel, Aufnahmen, Bilder und Darstellungen, Lehrmaterialien und ähnliche Zusatzinformationen in die Suche miteinbezogen werden können und damit die Diagnose und Bewertung deutlich erleichtert werden kann. Dies wird insbesondere durch die einfache Verwendung eines Datenmodells ermöglicht, das auf einer großen Datenbasis gewonnen wurde, die im täglichen Betrieb eines Spitals angefallen ist. Ebenso ist es auch möglich, dass eine Suche bzw Bewertung aufgrund eines Abfragebilds auf Grundlage einer breiten Datenbasis durchgeführt wird, die einzelnen der Datenbasis zugrunde liegenden Datensätze jedoch nicht angezeigt werden brauchen, da insbesondere ausschließlich auf andere Datensätze zugegriffen werden kann.

Eine weitere bevorzugte Ausführungsform der Erfindung verwendete 2D, 3D oder höherdimensionale Aufnahmen, beispielsweise radiologische Aufnahmen, CT-Aufnahmen oder MR-Aufnahmen, um dem Benutzer visuell ähnliche medizinisch relevante Fälle einer spitalsinternen Datenbank zur Verfügung zu stellen.

Eine weitere Ausführungsform der Erfindung verwendet für das Training die zur Verfügung stehende semantische Information, die einzelnen diagnostischen Berichten zugeordnet ist. Ebenso kann eine automatisierte Volltextsuche in medizinischer Literatur verwendet werden.

Eine weitere bevorzugte Ausführungsform der Erfindung betrifft ein System gemäss Anspruch 13 zur, insbesondere gleichzeitigen, Suche von medizinischen Bilddaten, medizinischen Text-Informationen und sematischer Information ausgehend von einer Suchanfrage eines Benutzers bestehend aus einem Bild und einem interessierenden Bereich. Der interessierende Bereich kann auch das gesamte Bild umfassen. Das System ermittelt als Ergebnis der Suchanfrage eine Rangfolge relevanter Datensätze. Relevantere oder dem Bild ähnlichere Datensätze werden höher gereiht als weniger relevante oder weniger ähnliche Datensätze. Ublicherweise bestehen Datensätze aus:
- medizinischen Bilddaten wie beispielsweise, aber nicht ausschließlich, computertomographische oder kernspintomographische Bilddaten,
- medizinischer Text-Information wie beispielsweise, aber nicht ausschließlich, radiologische Berichte, medizinische Webseiten, Publikationen, Literatur, didaktische Informationsquellen oder anderem Material,
- semantischen Informationen wie beispielsweise, aber nicht ausschließlich, gekennzeichnete Teilbereiche von Untersuchungsbildern und semantische Informationen aus klinischen Texten und

Zur Durchführung der einzelnen Operationen verfügt das System über eine Indizierungseinheit, eine Lerneinheit und eine Sucheinheit.

Bevorzugt werden eine Indizierungseinheit und eine Lerneinheit mit Daten aus verschiedenen Bereichen trainiert, wie (1) medizinischen Bilddaten wie beispielsweise, aber nicht ausschließlich, computertomographische oder kernspintomographische Bilddaten, und gleichzeitig (2) Bilddaten aus Dokumenten wie beispielsweise, aber nicht nur, Webseiten, Publikationen, Literatur, didaktische Informationsquellen oder anderes Material und gleichzeitig (3) semantischen Informationen.

Bevorzugt werden die Indizierungseinheit und die Lerneinheit zusätzlich trainiert werden mit patientenspezifischen Daten wie radiologischen Berichten und semantischer Information aus radiologischen Berichten.

Besonders vorteilhaft ist es, wenn die Lerneinheit und die Indizierungseinheit semantische Kennzeichnungen oder Text-Informationen aus der Domäne als zusätzliche Kosten-Terme im Training und Indizieren von Daten der anderen Domäne verwendet.

Weiters ist es vorteilhaft, wenn die Lerneinheit implizit auf Teilbereiche von Bilddaten, die semantisch sinnvoll sind fokussiert, wie Teilbilder ausgehend von Daten der anderen Domäne wie computertomographische oder kernspintomographe Volumen.

Auch ist es vorteilhaft, wenn zusätzlich die Lerneinheit den Maßstab von Bilddaten in der einen Domäne basierend auf bekannten Maßstäben in der anderen Domäne bestimmen kann, auch wenn die physikalische Größe oder Auflösung in der ersten Domäne unbekannt ist. Die Indizierungseinheit indiziert zudem die ermittelten Maßstäbe gemeinsam mit den Bilddaten.

Schließlich ist es von Vorteil, wenn die Lerneinheit ein CNN, welches Blöcke in einen gemeinsamen räumlichen Bezugsrahmen abbildet, trainieren kann, wobei das resultierende Modell verwendet werden kann, um individuelle Blöcke in den Bezugsrahmen abzubilden, aber auch um gesamte Volumen in diesen Bezugsrahmen abzubilden und derart die Bilddaten zu registrieren.

Weiters kann vorteilhaft vorgesehen sein, dass die Lerneinheit eine Lage, insbesonderer bestimmter zu ermittelnder Bildabschnitte, mittels CNNs abschätzen kann, wobei zusätzlich die Indizierungseinheit die abgeschätzte Lage gemeinsam mit den Bilddaten indiziert, oder wobei die Sucheinheit automatisch die Lage eines Abfragebilds und die Positionen im Abfragebild in einem anatomischen Referenzbezugssystem bestimmt, wodurch das Anzeigen und die weitere Verwendung, beispielsweise für eine Anreicherung der Suchanfrage, von Referenzkoordinaten, oder Namen von anatomischen Strukturen, oder Illustrationen von anatomischen Strukturen als Ergebnis der Suchanfrage ermöglicht ist.

Weiters ist es von Vorteil, wenn die Lerneinheit unter Vorgabe eines Worts oder mehrerer Wörter Vorhersagen hinsichtlich der Wahrscheinlichkeit dafür treffen kann, dass eines aus einer Reihe von semantischen Termen oder Identifikatoren vorliegt, die in einer Terminologie oder Ontologie wie RadLex, MESH, Snomed oder anderen gegeben sind.

Weiters ist es von Vorteil, wenn die Lerneinheit unter Vorgabe eines Worts oder mehrerer Wörter ein RNN trainiert, dessen/deren Wahrscheinlichkeit einen aus einer Reihe von semantischen Identifikatoren von medizinischen Fachbegriffen zu repräsentieren. Zusätzlich kann ein Score trainiert und vorhergesagt werden kann, der hinsichtlich dieser Identifikatoren z. B. "fehlend", "eher vorhanden" oder "vorhanden" angibt. Das Training kann vorteilhafterweise zusätzlich eine Prediction-cost-function mit zusätzlichen Kostenfunktion kombinieren, um Wort- oder Zeichensequenzen zu modellieren.

Die Teilbilder müssen nicht notwendigerweise Schnitte oder Blöcke sein und können auch nicht kubische oder nicht rechteckige Form aufweisen, z. B. Kreis- oder Kugelform.

Die Indizierungseinheit speichert vorzugsweise eine kompakte Repräsentation des Teilbilds, Schnittes oder Blocks jedes Bildes zusätzlich zu den Metadaten bzw Zusatzinformationen wie z. B. Patientenalter, Blockposition, semantische Information, in einer Datenstruktur, die für eine Schnelle Suche optimiert ist.

Vorteilhafterweise verwendet die Sucheinheit den Index verwendet, um die ähnlichsten Ergebnisse zu finden, wobei, falls gewünscht, Metadaten als Einschränkungen bei der Suche verwendet werden können.

Eine weitere vorteilhafte Weiterbildung der Erfindung sieht vor, dass die Datenstruktur in der Indizierungseinheit verwendet wird, um die indizierten Daten nach Strukturen zu durchsuchen, z. B. Finden von Clustern in den Daten oder Metadaten.

Weiters kann vorteilhafterweise vorgesehen sein, dass die Indizierungseinheit Indizes der Terme oder Text-Informationen in Form von Absatzvektoren oder Wortvektoren ablegt.

Die Indizierungseinheit erlernt vorteilhafterweise niedrigdimensional trennbare Filter und verarbeitet diese, um die Suchgeschwindigkeit zu erhöhen.

Besonders vorteilhaft kann vorgesehen sein, dass die Sucheinheit Indizes von Bilddaten und Textdaten und semantischen Daten verbindet.

Besonders vorteilhaft kann vorgesehen sein, dass die Sucheinheit ein Ergebnis von gereihten relevanten Datensätzen oder Fällen ermittelt, sodass relevantere oder ähnlichere Datensätze höher gereiht werden, als weniger relevante oder weniger ähnliche Datensätze,
wobei die Rangfolge von einer Projektion (representation function) festgelegt wird, die relevante semantische Ähnlichkeit berücksichtigt, auch wenn sie nur das Bild und eine ROI als Eingangsinformation erhält,
wobei die Projektion (representation function) auf CNNs und RNNs basiert, die trainiert werden von
   - einer an der Relativposition orientierte Kostenfunktion und/oder
   - einer halb-überwachte Kostenfunktion
   - einer schwach überwachte Kostenfunktion.

Weiters besteht auch die Möglichkeit, das erfindungsgemäße Verfahren vorteilhaft zu kaskadieren, um im Rahmen der Kaskade Informationen hinzuzugewinnen.

Es kann vorteilhaft vorgesehen sein, dass die Sucheinheit eine Suchanfrage eines Benutzers mit den Ergebnissen von Suchanfragen in verschiedenen Indizes von verschiedenen Domänen, beispielsweise mit Bildern oder Text-Information oder semantisch, anreichert, um eine nachfolgende Suchanfrage durchzuführen. Diese Suche kann relevante bzw relevantere Information liefern, insbesondere dann, wenn sie zur Erstellung weiterer Abfragen herangezogen wird.

Schließlich kann vorgesehen sein, dass die vom Benutzer oder einem weiteren System bereitgestellten Suchdaten im Browser vor der Übertragung der Daten anonymisiert werden.

Weitere Vorteile und Ausgestaltungen der Erfindung ergeben sich aus der Beschreibung und den beiliegenden Zeichnungen.

Besonders vorteilhafte, aber nicht einschränkend zu verstehende, Ausführungsbeispiele der Erfindung werden im Folgenden anhand der beiliegenden Zeichnungen schematisch dargestellt und unter Bezugnahme auf die Zeichnungen beispielhaft beschrieben:
**Fig. 1** zeigt schematisch die Erstellung eines Datensatzes in einer Datenbank.
**Fig. 2** zeigt schematisch eine Suchanfrage an die Datenbank aus **Fig. 1****.**
**Fig. 3** zeigt schematisch den Ablauf eines Mehr-Datenbank-Suchverfahrens

### Erstellung einer Datenbank mit medizinischen Bilddaten

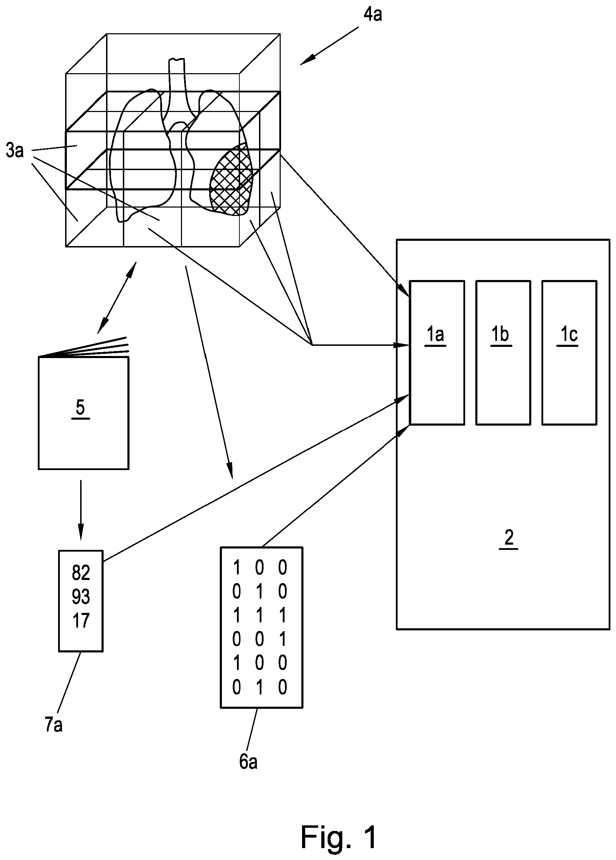

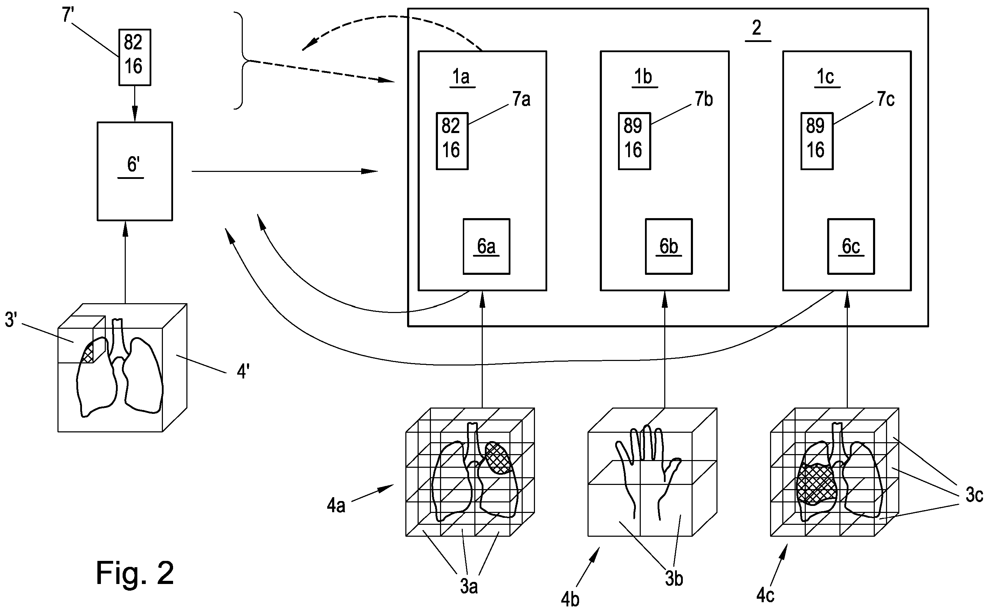

**Fig. 1** zeigt eine schematische Darstellung einer Datenbank 2 mit medizinischen Bilddaten, welche beispielsweise in einem Krankenhaus bei der Untersuchung von Patienten aufgenommen werden. Die Datenbank 2 ist auf einem Server im Krankenhaus oder im Internet zugänglich und beinhaltet in **Fig. 1** mehrere Datensätze 1a, 1b, 1c, wobei jeder Datensatz 1a, 1b, 1c Teilbilder 3a, 3b, 3c von zwei- oder mehrdimensionalen Ausgangsbildern 4a, 4b, 4c von Teilen des menschlichen Körpers umfasst (siehe **Fig. 2**).

Zur Erstellung der Datenbank 2 werden zunächst medizinische Bilddaten wie beispielsweise mit radiologischen Verfahren oder magnetischen Kernresonanzverfahren gewonnene Bilddaten als Ausgangsbilder 4a, 4b, 4c vorgegeben. Dabei handelt es sich beispielsweise um zweidimensionale Röntgenaufnahmen, sonographische Bilddaten oder Daten von bildgebenden Mikroskopieverfahren, aber auch um dreidimensionale röntgen- oder kernspintomographische Aufnahmen oder vierdimensionale Kontrastmittelbildsequenzen. In den Ausgangsbildern 4a, 4b, 4c werden systematisch große Zahlen von Teilbereichen, beispielsweise durch Verschieben eines Pixelrasters mit einer oder verschiedenen Rastergrößen entlang des jeweiligen Ausgangsbilds 4a, 4b, 4c, ausgewählt, wobei die ausgewählten Teilbereiche , als Teilbilder 3a, 3b, 3c in den Datensätzen 1a, 1b, 1c der Datenbank 2 hinterlegt werden. Durch die Auswahl von Teilbildern 3a, 3b, 3c, beispielsweise durch das systematische Verschieben eines Pixelrasters entlang des jeweiligen Ausgangsbilds 4a, 4b, 4c, ist auch deren Position im Ausgangsbild 4a, 4b, 4c bekannt und aus einem Ausgangsbild 4a, 4b, 4c werden jeweils z. B. 100.000 Teilbilder 3a, 3b, 3c ausgewählt. Ein Teilbild 3a, 3b, 3c kann aber auch einem gesamten Ausgangsbild 4a, 4b, 4c entsprechen.

Das in **Fig. 1** dargestellte Ausgangsbild 4a des Datensatzes 1a ist eine dreidimensionale computertomographische Aufnahme der Lunge eines Patienten. Dabei ist im Ausgangsbild 4a des Datensatzes 1a zu erkennen, dass ein Teilbereich des rechten Lungenflügels des Patienten Veränderungen aufweist. Die Ausgangsbilder 4b, 4c und die davon ausgewählten Teilbilder 3b, 3c der Datensätze 1b, 1c welche ebenfalls in der in **Fig. 1** schematisch dargestellten Datenbank 2 hinterlegt sind, sind in **Fig. 2** dargestellt.

Bei der Erstellung der Datenbank 2 wird zum speicherplatzsparenden Aufbau bzw. zur schnellen Durchsuchbarkeit der Datenbank 2 nach inhaltlich relevanten Teilbildern eine Projektion zur Gewinnung von Feature-Vektoren 6a, 6b, 6c aus den Teilbildern 3a, 3b, 3c erstellt. Die Projektion bildet dabei, insbesondere visuell oder semantisch, ähnliche Teilbilder 3a, 3b, 3c auf ähnliche Feature-Vektoren 6a, 6b, 6c ab und liefert bei Anwendung auf ein Teilbild 3a, 3b, 3c einen Feature-Vektor 6a, 6b, 6c, wobei insbesondere die Anzahl an Einträgen eines jeweiligen Feature-Vektors 6a, 6b, 6c geringer ist, als die Anzahl der Pixel der Teilbilder 3a, 3b, 3c.

Diese Reduzierung der Einträge eines Feature-Vektors 6a, 6b, 6c im Vergleich zur Anzahl der Pixel eines Teilbildes 3a, 3b, 3c führt vorteilhafterweise zu einer schnelleren Durchsuchbarkeit der Datenbank 2. Anstelle einer zeit- und rechenleistungsintensive Durchsuchung der Datenbank 2 nach ähnlichen Ausgangsbildern 4a, 4b, 4c oder Teilbildern 3a, 3b, 3c der Ausgangsbilder 4a, 4b, 4c ist eine Durchsuchung nach ähnlichen Feature-Vektoren 6a, 6b, 6c ausreichend, die die Teilbilder 3a, 3b, 3c oder Ausgangsbilder 4a, 4b, 4c repräsentieren. Weiters erlaubt diese Projektion Teilbilder 3a, 3b, 3c mit unterschiedlichem Aussehen mit denen aber ähnliche semantische Informationen wie z. B. die selbe Krankheit hinterlegt sind, auf ähnliche Feature-Vektoren abzubilden, worauf im Folgenden noch genauer eingegangen wird. So werden auch Teilbilder 3a, 3b, 3c auf ähnliche Feature-Vektoren 6a, 6b, 6c abgebildet, auf denen beispielsweise das abgebildete Gewebe Veränderungen mit unterschiedlichem visuellen Erscheinungsbild aufweist, die jedoch derselben Krankheit zuordenbar sind.

Zur Vorbereitung der Ausführung der Projektion wird ein neuronales Netzwerk, insbesondere ein Convolutional-Neural-Network, auf der Basis vorgegebener Lern-Teilbilder von einer Trainingseinheit erstellt. Die Datensätze 1a, 1b, 1c oder ein Teil der Datensätze 1a, 1b, 1c werden im Rahmen eines Metric-Learning-Verfahrens vom neuronalen Netzwerk dazu genutzt, die Projektion und das Erstellen der Feature-Vektoren 6a, 6b, 6c aus Lern-Teilbildern sowie einer zu erreichenden vorgegebenen Ähnlichkeit zwischen den Lern-Teilbildern zu erlernen.

Zum effektiven Erlernen der Projektion bzw. der Erstellung der Feature-Vektoren 6a, 6b, 6c werden dem betreffenden Metric-Learning-Verfahren [z.B. yang2006, schroff2015] dabei n-Tupel von Lern-Teilbildern oder Gruppen von Lern-Teilbildern einer oder mehrerer der folgenden Arten als ähnlich vorgegeben:
- Lern-Teilbilder, die zueinander geringfügig verschoben, rotiert, verzerrt oder gestreckt sind und ausgehend vom selben Ausgangsbild 4a, 4b, 4c erstellt werden und/oder
- Lern-Teilbilder, die ausgehend vom selben Teilbereich des Ausgangsbilds 4a, 4b, 4c erstellt werden, wobei zumindest eines der Lern-Teilbilder gegenüber dem Teilbereich des Ausgangsbilds 4a, 4b, 4c derart modifiziert wird, dass die Lern-Teilbilder unterschiedliches Rauschen und/oder unterschiedliche Bildintensität und/oder unterschiedlichen Kontrast aufweisen und/oder
- Lern-Teilbilder, von aus demselben Ausgangsbild 4a, 4b, 4c stammenden Teilbereichen, wobei die zu erreichende Ähnlichkeit zwischen den jeweiligen Lern-Teilbildern Bildern des n-Tupels vom räumlichen Abstand der betreffenden Teilbereiche im Ausgangsbild 4a, 4b, 4c abhängt, wobei insbesondere Lern-Teilbilder umso ähnlicher angesehen werden, je näher die betreffenden Teilbereiche im Ausgangsbild 4a, 4b, 4c beieinander liegen und/oder
- Erstellung einer komprimierten Repräsentation der in einem Teilbild 3a, 3b, 3c enthaltenen Informationen, beispielhaft dargestellt in Bengio, Yoshua, Aaron Courville, and Pascal Vincent. "Representation learning: A review and new perspectives." IEEE transactions on pattern analysis and machine intelligence 35.8 (2013): 1798-1828*,* Goodfellow, lan, et al. "Generative adversarial nets." Advances in neural information processing systems. 2014*;*
- Lern-Teilbildern vom selben Ausgangsbild 4a, 4b, 4c oder von unterschiedlichen Ausgangsbildern 4a, 4b, 4c, die aufgrund von mit den jeweiligen Ausgangsbildern 4a, 4b, 4c hinterlegten externen Merkmalen wie textuellen, numerischen oder semantischen Informationen als ähnlich anzusehen sind.

Im Anschluss an diese Lernphase wird die Projektion von einer Indizierungseinheit auf die Teilbilder 3a, 3b, 3c der Datensätze 1a, 1b, 1c und/oder auf eine Anzahl Teilbilder weiterer Datensätze angewendet und es wird dementsprechend für diese Teilbilder jeweils zumindest ein Feature-Vektor erstellt. Die derart erstellten Feature-Vektoren 6a, 6b, 6c werden, insbesondere verknüpft mit den Ausgangsbildern in den Datensätzen hinterlegt.

Ein für den Datensatz 1a der Datenbank 2 in **Fig. 1** beispielhaft dargestellter Feature-Vektor 6a beinhaltet 18 Einträge und umfasst eine wesentlich geringere Anzahl an Einträgen als der dreidimensionale Bildausschnitt jeweiliges eines Teilbilds 3a zeigt.

Die zur Erstellung der Datenbank 2 vorgegebenen einzelnen Ausgangsbilder 4a, 4b, 4c oder Teilbilder 3a, 3b, 3c können gegebenenfalls mit Zusatzinformationen 5 versehen sein, wobei die Zusatzinformationen 5 als Text-Informationen und/oder semantische Informationen und/oder numerische Informationen vorgegeben werden. Bei den Zusatzinformationen 5 kann es sich beispielsweise um medizinische Informationen handeln. Beispielsweise kann angegeben werden, dass ein Abschnitt des abgebildeten Körperteils Veränderungen aufweist, um welche Art von Veränderungen es sich dabei handelt oder von welcher Krankheit diese Veränderungen verursacht werden. Bei den Zusatzinformationen 5 kann es sich aber auch persönliche Informationen wie Alter und Geschlecht des Patienten handeln. Diese Zusatzinformationen 5 können optional ebenfalls in den Datensätzen 1a, 1b, 1c in der Datenbank 2 hinterlegt werden. Um derartige Zusatzinformationen 5 kompakt in der Datenbank 2 zu hinterlegen und semantisch relevante Zusammenhänge abzubilden, werden Text-Informationen oder numerische Informationen als Tags und/oder semantische Repräsentationen 7a, 7b, 7c und/oder semantische Informationen als semantische Repräsentationen 7a, 7b, 7c in der Indexdatenstruktur hinterlegt.

In dem in **Fig. 1** dargestellten Beispiel stehen als Zusatzinformationen 5 für den Datensatz 1a in der Datenbank 2 die Einträge in der Krankenakte des Patienten zur Verfügung. Dabei werden anstelle von Textinhalten der Krankenakte semantische Repräsentationen 7a der Einträge in der Krankenakte des Patienten für Alter und Geschlecht und Krankheitsbild im Datensatz 1a in der Datenbank 2 hinterlegt. Im Beispiel handelt es sich um einen 93-jährigen männlichen Patienten, bei dem Lungenkrebs diagnostiziert wurde. Als semantische Repräsentationen 7a werden in diesem Fall Zahlenkombinationen hinterlegt: "82" repräsentiert das Geschlecht des Patienten, "93" das Alter und "16" die diagnostizierte Krankheit. Komplexere Informationen wie z.B. umfangreichere Beschreibungen in klinischen Befunden können durch eigens trainierte neuronale Netzwerke wie z. B. Recurrent Neural Networks (RNNs), beispielhaft dargestellt in Sundermeyer, Martin, Ralf Schlüter, and Hermann Ney. "LSTM neural networks for language modeling." Thirteenth Annual Conference of the International Speech Communication Association. 2012*.,* oder Convolutional Neural Networks (CNNs) with attention mechanisms, beispielhaft dargestellt in Vaswani, Ashish, et al. "Attention Is All You Need." arXiv preprint arXiv:1706.03762 (2017*),* zu semantischen Codes umgewandelt, optional zusammen mit einer Gewichtung (wie z.B. "nicht vorhanden"/"leicht ausgeprägt"/"evtl vorhanden"/"definitiv vorhanden") und mit abgespeichert werden. Alternativ können ganze Absätze und Berichte durch neuronale Netzwerke wie z. B. Paragraph Vectors, wie beispielhaft dargestellt in Dai, Andrew M., Christopher Olah, and Quoc V. Le. "Document embedding with paragraph vectors." arXiv preprint arXiv:1507.07998 (2015*),* abgebildet und gespeichert werden.

Optional können derartige Zusatzinformationen 5 vom neuronalen Netzwerk zur Erstellung der Projektion, welche Teilbilder 3a, 3b, 3c auf Feature-Vektoren 6a, 6b, 6c abbildet, genutzt werden. Dabei wird die Projektion derart erstellt, dass Lern-Teilbilder als ähnlich vorgegeben werden, die von Ausgangsbildern 4a, 4b, 4c stammen oder Teilbildern 3a, 3b, 3c entsprechen, denen dieselbe Zusatzinformation 5 zugeordnet ist.

Im gezeigten Beispiel in **Fig. 1** werden beispielsweise zum Erlernen der Projektion bzw. der Erstellung der Feature-Vektoren 6a, 6b, 6c Lern-Teilbilder vorgegeben, auf denen das abgebildete Lungengewebe ähnliche Veränderungen zeigt und denen beispielsweise als Zusatzinformation 5 dieselbe Krankheitsbezeichnung zugeordnet ist. Dadurch werden Teilbilder 3a, 3b, 3c, auf denen Lungengewebe mit ähnlichen Veränderungen abgebildet ist und der jeweilige Patient an einer potentiell ähnlichen Krankheit leidet, auf ähnliche Feature-Vektoren 6a, 6b, 6c abgebildet.

Weiters ist die jeweilige Position der Teilbilder 3a, 3b, 3c eines Ausgangsbilds 4a, 4b, 4c bezogen auf den menschlichen Körper in den Datensätzen 1a, 1b, 1c in der Datenbank 2 hinterlegt. Die Informationen zur Position der Teilbilder 3a, 3b, 3c kann von einem neuronalen Netzwerk insbesondere dazu genutzt werden, eine Projektion zum Finden von Körperteilen mittels Feature-Vektoren 6a, 6b, 6c von Teilbildern 3a, 3b, 3c zu erlernen.

### Suchanfrage an die Datenbank

Zur Erstellung einer Suchanfrage an die Datenbank 2 wird zunächst vom Benutzer zumindest ein Abfragebild 3' aus zumindest einem zwei- oder mehrdimensionalen Untersuchungsbild 4' oder in einer Untersuchungsbildsequenz ausgewählt. Gegebenenfalls zur Verfügung stehende Zusatzinformationen 5 können aber zusätzlich zur Erstellung der Suchanfrage von einer Sucheinheit genutzt werden, sofern sie vorhanden sind.

Alternativ oder zusätzlich können zur Erstellung einer Suchanfrage auch vom Benutzer Text-Informationen wie z. B. ein Diagnosetext, numerische Informationen wie z. B. Alter oder Altersgruppe eines Patienten und/oder semantische Informationen wie z. B. eine Krankheit angegeben werden. Dabei kann von der Sucheinheit die Text-Informationen oder numerischen Informationen in Tags und/oder semantische Repräsentationen 7a, 7b, 7c umgewandelt werden. Anschließend wird nach Feature-Vektoren 6a, 6b, 6c gesucht, die ähnliche Tags und/oder semantische Repräsentationen 7a, 7b, 7c abbilden.

So ist es beispielsweise möglich, eine Suchanfrage nach Datensätzen zu erstellen, welche beispielsweise einem bestimmten Patientennamen oder einer bestimmten Krankheit zugeordnet sind. Erstellt ein Benutzer beispielsweise eine Suchanfrage mit "Handgelenksfraktur" als Text-Information, wird die vorgegebene Text-Information in eine semantische Repräsentation, im Beispiel die Zahlenkombination 53, umgewandelt und an die Datenbank 2 übermittelt. Als Ergebnis der Suche wird im Beispiel in **Fig. 2** in diesem Fall der Datensatz 1b ausgegeben, in dem eine dementsprechende semantische Repräsentation 7b hinterlegt ist.

Zur Erstellung einer Suchanfrage basierend auf Bildinformation an die Datenbank 2 wird zunächst für das Abfragebild 3' wie oben beschrieben ein oder mehrere Feature-Vektoren 6' des Abfragebilds 3' nach der erlernten Projektion ermittelt. Anschließend wird in der Datenbank 2 nach Datensätzen 1a, 1b, 1c mit Feature-Vektoren 6a, 6b, 6c gesucht, die unter Zugrundelegung einer vorgegebenen Metrik in der Nähe des Feature-Vektors 6' des Abfragebilds 3' liegen. Als Ergebnis der Suchanfrage werden, optional sortierte, Teilbilder 3a, 3b, 3c ausgegeben, die ein ähnliches Aussehen wie der Auswahlbereich aufweisen, optional zusammen mit den oder ersetzt durch die Datensätzen 1a, 1b, 1c.

Die Sortierung der Teilbilder des Ergebnisses ergibt sich aus der Ähnlichkeit der korrespondierenden Feature-Vektoren zu dem oder den Feature-Vektor(en) des Abfragebildes. Die Distanzen der Datensätze der 1a, 1b, 1c zu dem Abfragebild und somit eine optionale Sortierung können z.B. ermittelt werden durch die Akkumulierung der Distanzen der Ergebnisvektoren für jeden Datensatz durch die Anzahl der Ergebnisvektoren pro Datensatz innerhalb eines gewählten Ähnlichkeitsschwellwertes durch Analyse der räumlichen Konfiguration der den Ergebnisvektoren entsprechenden Teilbildern innerhalb der Ergebnisdatensätze durch Analyse der in der Datenbank zu den Ergebnisvektoren hinterlegten Zusatzinformationen.

Somit können Teilbilder 3a, 3b, 3c, welche beispielsweise ähnliche Krankheitsbilder abbilden, anhand der ihnen zugeordneten Feature-Vektoren 6a, 6b, 6c in der Datenbank 2 gefunden werden, ohne eine zeitaufwändige und rechenleistungsintensive Suche direkt nach Teilbildern 3a, 3b, 3c durchführen zu müssen. Eine vergleichsweise rasche Suche nach Feature-Vektoren 6a, 6b, 6c, die dem Feature-Vektor 6' des Abfragebilds 3' ähnlich sind, ist ausreichend, um ähnliche Teilbilder 3a, 3b, 3c und die damit assoziierten Ausgangsbilder 4a, 4b, 4c zu finden. Weiters erlaubt diese Vorgangsweise Feature-Vektoren 6a, 6b, 6c und korrespondierende Teilbilder 3a, 3b, 3c zu finden, die zwar visuell unterschiedlich sind, aber nach der von der Trainingseinheit erstellten Projektion semantisch relevant sind, da sie beispielsweise dem selben Krankheitsbild zugeordnet sind.

Weiters können optional vom Benutzer Kriterien vorgegeben werden, um beispielsweise die Anzahl an potentiellen Treffern bei der Suche in einer Datenbank 2 zu reduzieren. Beispielsweise können bei der Suche nach ähnlichen Feature-Vektoren 6a, 6b, 6c in der Datenbank 2 auch Suchergebnisse aufgrund der in den zugehörigen Datensätzen 1a, 1b, 1c in der Datenbank 2 hinterlegten Tags und/oder semantischen Repräsentationen 7a, 7b, 7c ausgeschlossen werden, wobei insbesondere Suchergebnisse unter vom Benutzer für die Tags und/oder semantischen Repräsentationen 7a, 7b, 7c vorgegeben Kriterien ausgeschlossen werden können. So kann z.B. nur nach Resultaten von Patienten des selben Geschlechts und der selben Altersgruppe gesucht werden.

Optional kann als Ergebnis der Suchanfrage eine Rangfolge der jeweils ermittelten Datensätzen 1a, 1b, 1c ausgegeben werden. Die ermittelten Datensätze 1a, 1b, 1c werden dabei von der Sucheinheit nach deren Ähnlichkeit oder Übereinstimmung zu den Tags und/oder semantischen Repräsentationen 7a, 7b, 7c der Suchanfrage und/oder nach deren, insbesondere durch den Abstand der betreffenden Feature-Vektoren 6a, 6b, 6c bestimmten, Ähnlichkeit zu zumindest einen Abfragebild 3' der Suchanfrage gereiht und die derart erstellte Rangfolge wird dem Benutzer angezeigt.

Optional können als Ergebnis der Suchanfrage die in den jeweiligen Datensätzen 1a, 1b, 1c als Tags hinterlegten Text-Informationen, numerischen Informationen und/oder die als semantische Repräsentationen 7a, 7b, 7c hinterlegten semantischen Informationen ausgegeben werden und/oder die Tags der zum Auswahlbereich ähnlichen Teilbilder 3a, 3b, 3c statistisch ausgewertet werden. Das so erhaltene statistische Ergebnis kann anschließend ausgegeben werden. Diese Statistik kann z. B. bei der Differentialdiagnose helfen, verschiedene, mit visuell ähnlichen Erscheinungen oder Veränderungen assoziierten, Krankheitsbilder zu gruppieren und dem Benutzer zu präsentieren. Weiters können z. B. vom Benutzer basierende auf den Suchergebnissen einfach Statistiken darüber erstellt werden, wie oft beispielsweise männliche oder weibliche Patienten unter einer bestimmten Krankheit leiden oder wie häufig eine bestimmte Altersgruppe von einer bestimmten Krankheit betroffen ist.

**Fig. 2** zeigt eine Datenbank 2 eines Krankenhauses mit drei darin hinterlegten Datensätzen 1a, 1b, 1c, welche Informationen zu Patienten bzw. Krankheitsfällen umfassen. Jeder der Datensätze 1a, 1b, 1c umfasst jeweils einen Feature-Vektor 6a, 6b, 6c welche jeweils ausgehend von in Ausgangsbildern 4a, 4b, 4c systematisch, beispielsweise mittels Verschieben eines Pixelrasters, ausgewählten Teilbildern 3a, 3b, 3c erstellt wurden. Die Ausgangsbilder 4a, 4b, 4c sind in **Fig. 2** dreidimensionale computertomographische Aufnahmen, wobei die Ausgangsbilder 4a, 4c Aufnahmen von Lungen zeigen und Ausgangsbild 4b eine Aufnahme einer Hand zeigt. Die in den Ausgangsbildern 4a, 4c dargestellten Lungenflügel weisen jeweils Veränderungen des Lungengewebes auf. Die im Ausgangsbild 4b dargestellte Hand weist eine Handgelenksfraktur auf.

Jeder Datensatz 1a, 1b, 1c in der Datenbank in **Fig. 2** umfasst weiters jeweils semantische Repräsentationen 7a, 7b, 7c. Im Beispiel repräsentieren Zahlenkombinationen das Geschlecht und die diagnostizierte Krankheit, welche jeweils in der Krankenakte des Patienten, mit dem die jeweiligen Ausgangsbilder 4a, 4b, 4c assoziiert sind, vermerkt sind. Im Beispiel repräsentiert 82 "männlich", 89 "weiblich", 16 die Diagnose "Lungenkrebs" und 53 "Handgelenksfraktur".

In dem in **Fig. 2** gezeigten Beispiel wird von einem Benutzer,, eine erste Suchanfrage an eine Datenbank 2 gestellt. Als Untersuchungsbild 4' wird eine dreidimensionale computertomographische Aufnahme einer Lunge vorgegeben. Im Untersuchungsbild 4' wird vom Benutzer ein Ausschnitt des linken Lungenflügels als Abfragebild 3' ausgewählt, da dieser Bereich des Lungenflügels Veränderungen aufweist. Um seine vorläufige Diagnose "Lungenkrebs" zu verifizieren, möchte der Benutzer als Ergebnis seiner Suchanfrage an die Datenbank 2 Datensätze erhalten, die Teilbilder von Lungengewebe mit ähnlichen Veränderungen enthalten und die zugehörigen Diagnosen mit der Diagnose vergleichen, die er vorläufig gestellt hat.

Das Abfragebild 3' dient zunächst allein zur Erstellung der ersten Suchanfrage, wobei Datensätze in der Datenbank 2 ermittelt werden sollen, in denen in der Datenbank 2 dem Abfragebild 3' ähnliche Teilbilder hinterlegt sind. Zur Erstellung der Suchanfrage wird zunächst, wie vorstehend beschrieben, nach der erlernten Projektion ausgehend vom Abfragebild 3' ein Feature-Vektor 6' des Abfragebilds 3' erstellt und als Suchanfrage an die Datenbank 2 übergeben, was in **Fig. 2** als durchgezogener Pfeil in Richtung der Datenbank 2 schematisch angedeutet ist. Anschließend werden in der Datenbank 2 nun jene Datensätze ermittelt, deren Feature-Vektoren dem Feature-Vektor 6' des Abfragebilds 3' ähnlich sind.

Beim in **Fig. 2** gezeigten Beispiel werden als Ergebnis der ersten Suchanfrage die Datensätze 1a und 1c ausgegeben; dieser Umstand ist durch durchgehende Pfeile aus der Datenbank 2 angedeutet. Die Feature-Vektoren 6a, 6c der Datensätze 1a, 1c wurden jeweils ausgehend von Teilbildern 3a, 3c eines Ausgangsbilds 4a, 4c erstellt, das jeweils eine dreidimensionale computertomographische Aufnahme eines Lungeflügels mit ähnlichen Veränderungen wie im Abfragebild 3' zeigt. Der im Datensatz 1b hinterlegte Feature-Vektor 6b weist keine ausreichende Ähnlichkeit mit dem Feature-Vektor 6' des Abfragebilds 3' auf, da der Feature-Vektor 6b basierend auf Teilbildern 3b eines Ausgangsbilds 4b erstellt wurde, welches einen Ausschnitt einer dreidimensionalen computertomographischer Aufnahme einer Hand eines Patienten zeigt, und wird daher nicht ausgegeben.

Zusätzlich werden die in den Datensätzen 1a, 1c hinterlegten semantischen Repräsentationen 7a, 7c ausgegeben, die jeweils die Zahlenkombination 16 für die Diagnose "Lungenkrebs" enthalten. Dem Benutzer stehen somit nun als Ergebnis seiner Suchanfrage die Datensätze 1a, 1c mit Aufnahmen von Lungengewebe, das ähnliche Veränderungen aufweist, wie das seines aktuellen Patienten und der jeweils gestellten Diagnose, im Beispiel "Lungenkrebs" zur Verfügung, um seine gestellte vorläufige Diagnose zu verifizieren.

In dem in **Fig. 2** gezeigten Beispiel stehen zur Erstellung der Suchanfragen auch semantische Repräsentationen 7' der Einträge in der Krankenakte des Patienten, auf dessen Untersuchungsbild 4' die Abfrage basiert, zur Verfügung. Die semantischen Repräsentationen 7' umfassen dabei die Zahlenkombinationen 82 für "männlich" und 16 für die vorläufige Diagnose "Lungenkrebs".

Der Benutzer möchte nun gezielt nach Datensätzen von männlichen Patienten suchen, bei denen Lungenkrebs diagnostiziert wurde, deren Lungengewebe ähnliche Veränderungen zeigt, wie das des aktuellen Patienten. Vom Benutzer wird daher eine zweite Suchanfrage an die Datenbank 2 gestellt, bei der zusätzlich zum Abfragebild 3' als Suchkriterien "männlich" und "Lungenkrebs" vom Benutzer vorgegeben werden. Zur Erstellung der zweiten Suchanfrage werden daher das Abfragebild 3' und die semantischen Repräsentationen 7' umfassend 82 für "männlich" und 16 für "Lungenkrebs" an die Datenbank 2 übermittelt, was in **Fig. 2** als strichlierter Pfeil in Richtung der Datenbank 2 schematisch dargestellt ist.

Bei der zweiten Suchanfrage wird nun in der Datenbank 2 nach Datensätzen mit Feature-Vektoren gesucht, die unter Zugrundelegung einer vorgegebenen Metrik in der Nähe des Feature-Vektors 6' des Abfragebilds 3' liegen, wobei Datensätze ausgeschlossen werden, deren semantische Repräsentationen 7' die Zahlenkombinationen 82 für "männlich" und 16 für "Lungenkrebs" nicht umfassen.

In dem in **Fig. 2** gezeigten Beispiel wird als Ergebnis der zweiten Suchanfrage der Datensatz 1a ausgegeben, was als strichlierter Pfeil aus der Datenbank 2 angedeutet ist. Der Datensatz 1a umfasst einen Feature-Vektor 6a, der dem Feature-Vektor 6' des Abfragebilds 3' ähnlich ist und zusätzlich umfassen die semantischen Repräsentationen 7a die Zahlenkombinationen 82 für "männlich" und 16 für "Lungenkrebs".

Der Datensatz 1c wird nicht als Ergebnis der zweiten Anfrage ausgegeben, obwohl der Feature-Vektor 6c, dem Feature-Vektor 6' des Abfragebilds 3' ähnlich ist, da die semantischen Repräsentationen 7c die Zahlenkombination 89 für "weiblich" umfasst und somit nicht mit allen Kriterien der zweiten Suchanfrage übereinstimmt. Dem Benutzer steht somit zur Verifikation seiner vorläufigen Diagnose der Datensatz 1a zur Verfügung, der Aufnahmen der Lunge eines männlichen Patienten mit Lungenkrebs enthält.

Alternativ kann das Ergebnis einer ersten Suchanfrage, z.b. "Lungenkrebs", verwendet werden um z.B. in Referenzdatenbanken, Webseiten, Datenbanken mit wissenschaftlichen Artikeln oder Krankenhausinformationssystemen nach relevanten Inhalten zu suchen. Weiter können durch die Zusatzinformation der Datensätze zur Position innerhalb des menschlichen Körpers Informationen im Zusammenhang mit der entsprechenden anatomischen Position ("Lunge links unten") oder dem entsprechenden Organ ("linker unterer Lungenlappen") ausgegeben werden.

Alternativ kann zur Erstellung einer Suchanfrage nach einem Untersuchungsbild 4' oder einer Untersuchungsbildsequenz ähnlichen Bildern durch Erstellung eines Schnitts ein dimensionsreduziertes und gegebenenfalls in seinen Abmessungen reduziertes Abfrageteilbild erstellt und dieses Abfrageteilbild für die Suchanfrage verwendet werden. Somit können beispielsweise höherdimensionalere Untersuchungsbilder 4' zur Erstellung einer Suchanfrage an eine niedrigdimensionalere Datenbank 2 verwendet werden. Ein zweidimensionaler Schnitt beispielsweise aus einer dreidimensionalen computertomographischen Aufnahme kann auf diese Weise als Abfrageteilbild für eine Suchanfrage in einer Datenbank 2 umfassend Datensätze mit Abbildungen aus wissenschaftlichen Artikeln genutzt werden.

Alternativ kann zur Erstellung einer Suchanfrage an eine Datenbank 2 von einem Benutzer z. B. ein zweidimensionales Abfragebild 3' aus einem wissenschaftlichen Artikel oder von einer Website, oder ein Ausschnitt davon, vorgegeben werden. Die abzufragende Datenbank 2 kann dabei Datensätze 1a, 1b, 1c mit zweidimensionalen oder dreidimensionalen Ausgangs- und/oder Teilbildern enthalten.

Eine derartige Suchanfrage an eine höherdimensionale Datenbank 2 ausgehend von einem niedrigerdimensionalen Abfragebild 3' ist möglich, wenn die Trainingseinheit beim Erstellen von Projektionen zur Gewinnung von Feature-Vektoren 6a, 6b, 6c aus Teilbildern 3a, 3b, 3c, darauf trainiert wurde, dass, insbesondere visuell oder semantisch, ähnliche Teilbilder 3a, 3b, 3c unabhängig von deren Teilbildformat wie beispielsweise Teilbilddimension oder -größe auf ähnliche Feature-Vektoren 6a, 6b, 6c abbilden.

Somit werden Teilbilder 3a, 3b, 3c, die von einer ersten Projektion, welche ausgehend von Lern-Teilbildern mit einer ersten Dimension erlernt wurde, auf ähnliche Feature-Vektoren 6a, 6b, 6c abgebildet werden auch von einer zweiten Projektion, die ausgehend von Lern-Teilbildern mit einer zweiten Dimension erlernt wurde, ebenfalls auf ähnliche Feature-Vektoren 6a, 6b ,6v abgebildet. Daher werden Strukturen, welche beispielsweise in einem dreidimensionalen Raum ähnlich ist, ist auch in einem Schnitt als ähnlich erkannt.

Weiters kann von einem Benutzer optional ein Abfragebild 3' mit unbekannter Position im menschlichen Körper vorgegeben und als Suchanfrage an die Datenbank 2 übermittelt werden. In diesem Fall wird zunächst die gesuchte Position im menschlichen Körper ermittelt und in der Datenbank 2 nach Feature-Vektoren 6a, 6b, 6c von Teilbildern 3a, 3b, 3c gesucht, für die dieselbe Position bezogen auf den menschlichen Körper in deren Datensätzen 1a, 1b, 1c hinterlegt ist oder deren Position einen vom Benutzer vorgegebenen Abstandsschwellenwert zur gesuchten Position nicht überschreitet. Als Ergebnis der Suchanfrage werden Teilbilder 3a, 3b, 3c ermittelt, die einen räumlich ähnlichen Ausschnitt eines menschlichen Körpers zeigen, wie das Abfragebild 3'.

Optional können die einer Suchanfrage zugrunde liegenden Untersuchungsbilder 4' und/oder Untersuchungsbildsequenzen und/oder Text-Informationen und/oder numerischen Informationen und/oder semantischen Informationen vor der Übermittlung der Suchanfrage durch den Benutzer an die Datenbank 2 anonymisiert werden. Auf diese Weise kann vom Benutzer sichergestellt werden, dass die persönlichen Daten eines Patienten beim Stellen einer Suchanfrage nicht an die Datenbank 2 übermittelt werden. Dies kann z.B. nach den DICOM PS3.15 2013 Anonymisierungsrichlinien erfolgen.

Weiters können optional auf eine Suchanfrage eines Benutzers in der Datenbank 2 Gruppen von Teilbildern 3a, 3b, 3c und/oder Text-Informationen und/oder numerischen Informationen und/oder semantischen Informationen mit ähnlichen Feature-Vektoren 6a, 6b, 6c erstellt und ausgegeben und gegebenenfalls Zusatzinformationen 5 hinsichtlich dieser Gruppen, und/oder benachbarte Gruppen von Teilbildern 3a, 3b, 3c und/oder Text-Informationen und/oder numerischen Informationen und/oder semantischen Informationen mit ähnlichen Feature-Vektoren 6a, 6b, 6c ermittelt und ausgegeben werden und gegebenenfalls Zusatzinformationen 5 hinsichtlich dieser Gruppen ausgegeben werden.

### Mehr-Datenbank-Verfahren (Kaskadensuche)

Eine Ausführungsform der Erfindung bietet die Möglichkeit, ein mehrstufiges Suchverfahren aufeinanderfolgend in mehreren Datenbanken durchzuführen. Zunächst wird, wie oben beschrieben, eine erste Suchanfrage an eine Datenbank 2 übermittelt. Die als Ergebnis dieser ersten Suchanfrage an die Datenbank 2 ermittelten Suchergebnisse, insbesondere Teilbilder 3a, 3b, 3c, Gruppeninformationen, Textinformationen, numerische Informationen und/oder semantische Informationen, werden anschließend zur Erstellung zumindest einer weiteren Suchanfrage verwendet und diese weitere Suchanfrage wird an zumindest eine weitere Datenbank 2a übermittelt.

Zur Erstellung der zumindest einen weiteren Suchanfrage an die zumindest eine weitere Datenbank 2a können alternativ oder zusätzlich weitere als Ergebnis der ersten Suchanfrage an die Datenbank 2 ausgegebene Informationen, insbesondere ausgegebene statistische Ergebnisse und/oder Ausgangsbilder 4a, 4b, 4c und/oder ähnliche Teilbilder 3a, 3b, 3c verwendet werden, wobei die Dimension der ausgegebenen Ausgangsbilder 4a, 4b, 4c und/oder der ausgegebenen Teilbilder 3a, 3b, 3c für die Suchanfrage in der zumindest einen weiteren Datenbank 2a, beispielsweise eine Literaturdatenbank, gegebenenfalls reduziert wird.

So ist es beispielsweise möglich, ausgehend von ermittelten dreidimensionalen Teilbildern 3a, 3b, 3c eine weitere Suchanfrage zu erstellen und an eine Literaturdatenbank, welche nur zweidimensionale Aufnahmen enthält, zu übermitteln, wobei als Ergebnis beispielsweise Datensätze mit zweidimensionalen Abbildungen aus wissenschaftlichen Publikationen ausgegeben werden.

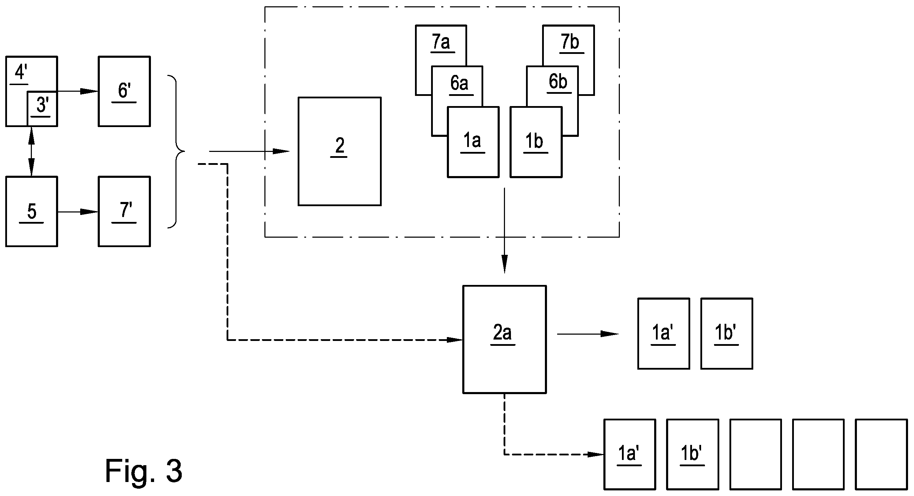

**Fig. 3** zeigt schematisch den Ablauf eines derartigen Suchverfahrens zur Suche in der in **Fig. 1** und **Fig. 2** gezeigten Datenbank 2, welche Datensätze 1a, 1b, 1c mit im Rahmen von Untersuchungen an Patienten ermittelten Informationen umfasst, und in einer weiteren Datenbank 2a, welche weitere Datensätze 1a', 1b' mit Informationen aus der Fachliteratur umfasst.

Zunächst wird von einem Benutzer, einem im Krankenhaus beschäftigten Arzt, zur Erstellung einer ersten Suchanfrage an die Datenbank 2 ein Untersuchungsbild 4' vorgegeben und ein Abfragebild 3' darin ausgewählt. Zusätzlich gibt der Benutzer im gezeigten Beispiel zur Erstellung der ersten Suchanfrage an die Datenbank 2 Zusatzinformationen 5 als Suchkriterien an, was aber keinesfalls zwingend notwendig ist. Ausgehend vom Abfragebild 3' wird nach der wie vorstehend beschrieben erstellten Projektion ein Feature-Vektor 6' des Abfragebilds 3' erstellt, die Zusatzinformationen 5 werden in semantische Repräsentationen 7' umgewandelt und beides an die Datenbank 2 als erste Suchanfrage übermittelt, was als durchgehender Pfeil angedeutet ist.

Als Ergebnis der ersten Suchanfrage werden von der Datenbank 2 die Datensätze 1a, 1b ausgegeben, welche Feature-Vektoren 6a, 6b umfassen, die in der Nähe des Feature-Vektors 6' des Abfragebilds 3' liegen, und die semantische Repräsentationen 7a, 7b umfassen, welche den vom Benutzer vorgegebenen semantische Repräsentationen 7' ähnlich sind.

Im Beispiel in **Fig. 3** werden die in der ersten Suchanfrage an die Datenbank 2 ermittelten Datensätze 1a, 1b, anschließend zur Erstellung einer weiteren Suchanfrage verwendet. Dazu werden Schnitte durch die dreidimensionalen Teilbilder 3a, 3b erstellt und zur Erstellung der weiteren Suchanfrage verwendet, welche an die weitere Datenbank 2a übermittelt wird. Als Ergebnis der zweiten Suchanfrage werden von der weiteren Datenbank 2a die weiteren Datensätze 1a', 1b' ausgegeben, welche zweidimensionale Abbildungen aus wissenschaftlichen Publikationen enthalten, deren Feature-Vektoren in der Nähe der Feature-Vektoren 6a, 6b der als Ergebnis der ersten Suchanfrage ermittelten Datensätze 1a, 1b liegen.

Somit stehen dem Benutzer im Beispiel in **Fig. 3** nun insgesamt vier Datensätze zur Verfügung, welche seinem gewählten Abfragebild 3' ähnlich sind, wobei im Beispiel die Datensätze 1a, 1b der Datenbank 2 dreidimensionale Teilbilder 3a, 3b bzw. Ausgangsbilder 4a, 4b von Patienten des Krankenhauses und die weiteren Datensätze 1a', 1b' der weiteren Datenbank 2a zweidimensionale Abbildungen aus einem wissenschaftlichen Fachjournal enthalten.

Zusätzlich können zur Erstellung der zweiten Suchanfrage auch beispielsweise in den in der ersten Suchanfrage ermittelten Datensätzen 1a, 1b hinterlegte semantische Repräsentationen 7a, 7b genutzt werden. In diesem Fall werden in der weiteren Datenbank 2a Datensätze ermittelt, die semantische Repräsentationen umfassen, welche den semantischen Repräsentationen 7a, 7b ähnlich sind.

Optional können bei einem derartigen mehrstufigen Suchverfahren auch einzelne Datensätze 1a, 1b, 1c der Datenbank 2 ausschließlich für die Bildung einer Projektionsfunktion herangezogen werden, dem Benutzer jedoch nicht als Resultate von Abfragen zur Ansicht zur Verfügung gestellt werden.

Dieser Fall ist in **Fig. 3** durch die strichpunktierte Umrandung der Datenbank 2 angedeutet. Ein Benutzer, beispielsweise ein Arzt mit einer eigenen Praxis, hat zwar keinen direkten Zugriff auf die Datensätze 1a, 1b, 1c in der Datenbank 2, kann die Datenbank 2 aber zur Erstellung einer mehrstufigen Suchanfrage nutzen. In diesem Fall wird vom Benutzer ausgehend vom Untersuchungsbild 4' zur Erstellung einer ersten Suchanfrage ein Abfragebild 3' ausgewählt. Für das Abfragebild 3' wird ein Feature-Vektor 6' des Abfragebilds 3' ermittelt und an die Datenbank 2 übermittelt.

Als Ergebnis werden, wie zuvor beschrieben, die Datensätze 1a, 1b ermittelt deren Teilbilder 3a, 3b ein ähnliches Aussehen wie das ausgewählte Abfragebild 3' haben. Die Datensätze 1a, 1b werden dem Benutzer jedoch nicht angezeigt, sondern ausschließlich zur Erstellung einer weiteren Suchanfrage verwendet, welche an die weitere Datenbank 2a übermittelt wird. Der Benutzer erhält schließlich als Ergebnis seiner Suchanfrage die weiteren Datensätze 1a', 1b' der weiteren Datenbank 2a, deren Teilbilder dem ausgewählten Abfragebild 3' und den Teilbildern 3a, 3b der Datensätze 1a, 1b ähnlich sind.

Der Fall, dass der Benutzer direkt eine Suchanfrage an die weitere Datenbank 2a übermittelt, ist in **Fig. 3** durch die strichlierten Pfeile dargestellt. In diesem Fall wird vom Benutzer eine Suchanfrage direkt an die weitere Datenbank 2a übermittelt und als Ergebnis der Suchanfrage werden in der weiteren Datenbank 2a fünf Datensätze ermittelt und dem Benutzer angezeigt. Die angezeigten Suchergebnisse beinhalten in diesem Fall auch weniger relevante Datensätze, da im Vergleich zur mehrstufigen Suche das Kriterium der Ähnlichkeit zu den Datensätzen 1a', 1b' der Datenbank 2 wegfällt. Dies zeigt, dass ein mehrstufiges Suchverfahren präzisere Suchergebnisse liefert.

Optional können bei der Erstellung der Datenbank 2 in den Datensätzen 1a, 1b, 1c auch Größenangaben betreffend die Pixelabmessungen oder Voxelabmessungen der Ausgangsbilder 4a, 4b, 4c oder Teilbilder 3a, 3b, 3c hinterlegt werden und/oder die Pixelabmessungen oder Voxelabmessungen für die Ausgangsbilder 4a, 4b, 4c oder Teilbilder 3a, 3b, 3c vorgegeben werden.

Zur Vorgabe der Pixelabmessungen oder Voxelabmessungen wird nach ähnlichen, insbesondere vom selben Körperteil herrührenden, Referenz-Ausgangs- oder Referenz-Teilbildern mit bekannten Pixel- oder Voxelabmessungen gesucht und anschließend durch Bildvergleich eine Skalierung gesucht, mit der das Ausgangs- oder Teilbild mit dem Referenz-Ausgangs- oder Referenz-Teilbild optimal in Übereinstimmung gebracht werden kann und ausgehend von dieser Skalierung und den bekannten Pixelabmessungen oder Voxelabmessungen des Referenz-Ausgangs- oder Referenz-Teilbilds werden die Pixelabmessungen oder Voxelabmessungen des Ausgangsbilds 4a, 4b, 4c oder Teilbilds 3a, 3b, 3c ermittelt und in der Datenbank 2 abgespeichert.

Auf ähnliche Weise kann bei Bedarf zu einem Abfragebild 3', zu dem keine Korrespondenz zwischen Pixel-/Voxelgröße und physikalischen Maßeinheiten wie z.B. mm bekannt ist, eine solche Korrespondenz geschätzt werden, indem eine Suchanfrage an die Datenbank 2 erstellt wird und Korrespondenzen zwischen Pixel-/Voxelgröße und physikalischer Maßeinheit der als Ergebnis der Suchanfrage ermittelten Referenz-Ausgangs- oder Referenz-Teilbilder verwendet werden, um die Korrespondenz im Abfragebild 3' zu schätzen.

## Patentansprüche

1. Verfahren zum Erstellen einer medizinischen Bilddatenbank,
a) wobei Datensätze (1a, 1b, 1c) erstellt werden, die Teilbilder (3a, 3b, 3c) von zwei- oder mehrdimensionalen Ausgangsbildern (4a, 4b, 4c) von Teilen des menschlichen Körpers umfassen, und wobei
- für jedes Teilbild (3a, 3b, 3c) eines Ausgangsbilds (4a, 4b, 4c) die jeweilige Position in einem anatomischen Referenzatlas bekannt ist, und
- die einzelnen Ausgangsbilder (4a, 4b, 4c) oder Teilbilder (3a, 3b, 3c) gegebenenfalls mit textuellen, numerischen oder semantischen Zusatzinformationen (5a, 5b, 5c) versehen sind, und
- ein Teilbild (3a, 3b, 3c) auch einem gesamten Ausgangsbild (4a, 4b, 4c) entsprechen kann, wobei
b) eine Projektion zur Gewinnung von Feature-Vektoren (6a, 6b, 6c) aus den Teilbildern (3a, 3b, 3c) erstellt wird,
- die, insbesondere visuell oder semantisch, ähnliche Teilbilder (3a, 3b, 3c) auf Feature-Vektoren (6a, 6b, 6c) mit geringem Abstand abbildet, und
- wobei zur Vorbereitung der Ausführung der Projektion ein neuronales Netzwerk, insbesondere ein Convolutional-Neural-Network, auf der Basis vorgegebener Lern-Teilbilder erstellt wird, wobei die Datensätze (1a, 1b, 1c) oder ein Teil der Datensätze (1a, 1b, 1c) im Rahmen eines Metric-Learning-Verfahrens vom neuronalen Netzwerk dazu genutzt werden, die Projektion und das Erstellen der Feature-Vektoren (6a, 6b, 6c) einer komprimierten Repräsentation der in einem Lern-Teilbild enthaltenen Informationen aus Lern-Teilbildern oder Gruppen von Lern-Teilbildern sowie einer zu erreichenden vorgegebenen Ähnlichkeit zwischen den Lern-Teilbildern zu erlernen, wobei dem betreffenden Metric-Learning-Verfahren eine oder mehrere der folgenden Vorgaben zugrunde gelegt werden:
- Vorgabe von n-Tupeln von Lern-Teilbildern oder Gruppen von Lern-Teilbildern als ähnlich, die zueinander geringfügig verschoben, rotiert, verzerrt oder gestreckt sind und ausgehend vom selben Ausgangsbild (4a, 4b, 4c) erstellt wurden, und/oder
- Vorgabe von n-Tupeln von Lern-Teilbildern oder Gruppen von Lern-Teilbildern als ähnlich, die ausgehend vom selben Teilbereich (3a, 3b, 3c) des Ausgangsbilds (4a, 4b, 4c) erstellt werden, wobei zumindest eines der Lern-Teilbilder gegenüber dem Teilbereich des Ausgangsbilds (4a, 4b, 4c) derart modifiziert wird, dass die Lern-Teilbilder unterschiedliches Rauschen und/oder, unterschiedliche Bildintensität und/oder unterschiedlichen Kontrast aufweisen, und/oder
- Vorgabe von n-Tupeln von aus demselben Ausgangsbild oder Gruppen von Ausgangsbildern (4a, 4b, 4c) stammenden Teilbereichen als Lern-Teilbilder, wobei die zu erreichende Ähnlichkeit zwischen den jeweiligen Lern-Teilbildern Bildern des n-Tupels vom räumlichen Abstand der betreffenden Teilbereiche im Ausgangsbild (4a, 4b, 4c) abhängt, wobei insbesondere Lern-Teilbilder umso ähnlicher angesehen werden, je näher die betreffenden Teilbereiche im Ausgangsbild (4a, 4b, 4c) beieinander liegen, und/oder
- Vorgabe von Lern-Teilbildern oder Gruppen von Lern-Teilbildern vom selben Ausgangsbild (4a, 4b, 4c) oder von unterschiedlichen Ausgangsbildern (4a, 4b, 4c), die aufgrund von mit den jeweiligen Ausgangsbildern (4a, 4b, 4c) hinterlegten externen Merkmalen von den textuellen, numerischen oder semantischen Zusatzinformationen als ähnlich anzusehen sind,
c) wobei auf die Teilbilder (3a, 3b, 3c) der Datensätze (1a, 1b, 1c) und/oder auf eine Anzahl weiterer Teilbilder weiterer Datensätze die Projektion angewendet wird und dementsprechend für diese Teilbilder jeweils zumindest ein Feature-Vektor erhalten wird, und
d) wobei die derart erstellten Feature-Vektoren, verknüpft mit den Ausgangsbildern und/oder Datensätzen (1a, 1b, 1c) oder weiteren Datensätzen, in einer Indexdatenstruktur hinterlegt werden,
wobei die jeweilige Position der Teilbilder (3a, 3b, 3c) eines Ausgangsbilds (4a, 4b, 4c) bezogen auf den menschlichen Körper in dem anatomischen Referenzatlas ermittelt wird, und, wobei die Informationen zur Position der Teilbilder (3a, 3b, 3c) von einem neuronalen Netzwerk dazu genutzt werden, eine Projektion zur Schätzung der Positionen von Teilbildern (3a, 3b, 3c) zu erlernen,
- wobei die Projektion gelernt wird mit der Zielfunktion, dass durch Abbildung von Paaren oder Gruppen von Teilbildern (3a, 3b, 3c) die räumliche Konstellation der Paare/Gruppen vor und nach der Projektion ähnlich ist und
- wobei die Projektion basierend auf einer bekannten Abbildung von Teilbildern auf Positionen gelernt wird
- wobei zusätzlich zu den Feature-Vektoren (6a, 6b, 6c) die gelernte oder bekannte Positionsinformation in der Datenbank abgespeichert wird
- wobei bei Vorliegen einer Suchanfrage die gesuchte Position im Körper ermittelt wird und in der Datenbank (2) nach Feature-Vektoren (6a, 6b, 6c) von Teilbildern (3a, 3b, 3c) gesucht wird, für die dieselbe Position in deren Datensätzen (1a, 1b, 1c) hinterlegt ist oder deren Position einen vom Benutzer vorgegebenen Abstandsschwellenwert zur gesuchten Position nicht überschreitet.

2. Verfahren nach Anspruch 1, **dadurch gekennzeichnet, dass** in den Datensätzen (1a, 1b, 1c) Zusatzinformationen (5) hinterlegt werden, wobei die Zusatzinformationen (5) als Text-Informationen und/oder semantische Informationen und/oder numerische Informationen vorgegeben werden, und
dass optional bei Vorliegen von Text-Informationen oder numerischen Informationen die Text-Informationen oder numerischen Informationen als Tags und/oder semantische Repräsentationen (7a, 7b, 7c) in der Datenbank (2) hinterlegt werden, und
dass optional die Zusatzinformationen (5) vom neuronalen Netzwerk zur Erstellung der Projektion genutzt werden, wobei die Projektion derart erstellt wird, dass Lern-Teilbilder als ähnlich vorgegeben werden, die von Ausgangsbildern (4a, 4b, 4c) stammen oder Teilbildern (3a, 3b, 3c) entsprechen, denen dieselbe Zusatzinformation (5) zugeordnet ist.

3. Verfahren nach einem der Ansprüche 1 bis 2, **dadurch gekennzeichnet,**
- **dass** zur Erstellung einer Suchanfrage vom Benutzer Text-Informationen, numerische Informationen und/oder semantische Informationen angegeben werden,
- **dass** optional die Text-Informationen oder numerischen Informationen in Tags und/oder semantische Repräsentationen (7a, 7b, 7c) umgewandelt werden
- **dass** bei Vorliegen einer Suchanfrage in der Datenbank (2) nach Feature-Vektoren (6a, 6b, 6c) gesucht wird, die ähnliche Tags und/oder semantische Repräsentationen (7a, 7b, 7c) besitzen.

4. Verfahren nach einem der Ansprüche 1 bis 2, **dadurch gekennzeichnet,**
- **dass** zur Erstellung einer Suchanfrage zumindest ein Abfragebild (3'), insbesondere eines interessierenden Bereichs, aus zumindest einem zwei- oder mehrdimensionalen Untersuchungsbild (4') oder in einer Untersuchungsbildsequenz ausgewählt wird,
- für das Abfragebild (3') nach Schritt c des Anspruchs 1 ein Feature-Vektor nach der erlernten Projektion ermittelt wird,
- in der Datenbank (2) nach Datensätzen (1a, 1b, 1c) mit Feature-Vektoren (6a, 6b, 6c) gesucht wird, die unter Zugrundelegung einer vorgegebenen Metrik in der Nähe des Feature-Vektors (6') des Abfragebilds (3') liegen, und
als Ergebnis der Suchanfrage Datensätze (1a, 1b, 1c) ausgegeben werden, deren Teilbilder (3a, 3b, 3c) ein ähnliches Aussehen wie der Auswahlbereich aufweisen oder die semantisch relevant sind, und
optional wobei zur Suche ähnlicher Bilder für ein Untersuchungsbild (4') oder eine Untersuchungsbildsequenz durch Erstellung eines Schnitts ein dimensionsreduziertes und gegebenenfalls in seinen Abmessungen reduziertes Abfrageteilbild erstellt wird und dieses Abfrageteilbild für die Suchanfrage verwendet wird.

5. Verfahren nach einem der Ansprüche 3 oder 4, **dadurch gekennzeichnet, dass** bei der Suche nach ähnlichen Feature-Vektoren (6a, 6b, 6c) in der Datenbank (2) Suchergebnisse aufgrund der in den zugehörigen Datensätzen (1a, 1b, 1c) in der Datenbank (2) hinterlegten Tags und/oder semantischen Repräsentationen (7a, 7b, 7c) ausgeschlossen werden,
insbesondere, dass Suchergebnisse unter vom Benutzer für die Tags und/oder semantischen Repräsentationen (7a, 7b, 7c) vorgegeben Kriterien ausgeschlossen werden.

6. Verfahren nach einem der Ansprüche 3 bis 5, **dadurch gekennzeichnet,**
**dass** als Ergebnis der Suchanfrage eine Rangfolge der jeweils ermittelten Datensätze (1a, 1b, 1c) nach deren Ähnlichkeit oder Übereinstimmung zu den Tags und/oder semantischen Repräsentationen (7a, 7b, 7c) der Suchanfrage und/oder nach deren, und/oder durch den Abstand der betreffenden Feature-Vektoren (6a, 6b, 6c) bestimmten, Ähnlichkeit zu zumindest einen Abfragebild (3') der Suchanfrage erstellt und ausgegeben wird.

7. Verfahren nach einem der Ansprüche 3 bis 6, **dadurch gekennzeichnet,**
- **dass** als Ergebnis der Suchanfrage die in den jeweiligen Datensätzen (1a, 1b, 1c) als Tags hinterlegten Text-Informationen, numerischen Informationen und/oder die als semantische Repräsentationen (7a, 7b, 7c) hinterlegten semantischen Informationen ausgegeben werden und/oder
- **dass** die Tags der zum Auswahlbereich ähnlichen Teilbilder (3a, 3b, 3c) statistisch ausgewertet werden und das statistische Ergebnis ausgegeben wird.

8. Verfahren nach einem der Ansprüche 3 bis 7, **dadurch gekennzeichnet, dass** die der Suchanfrage zugrunde liegenden Untersuchungsbilder (4') und/oder Untersuchungsbildsequenzen und/oder Text-Informationen und/oder numerischen Informationen und/oder semantischen Informationen vor der Übermittlung der Suchanfrage durch den Benutzer an die Datenbank (2) anonymisiert werden.

9. Verfahren nach einem der Ansprüche 3 bis 8, **dadurch gekennzeichnet,**
- **dass** ohne eine Suchanfrage direkt aufgrund der in der Datenbank vorhandenen Daten in der Datenbank (2) Gruppen von Teilbildern (3a, 3b, 3c) und/oder Text-Informationen und/oder numerischen Informationen und/oder semantischen Informationen mit ähnlichen Feature-Vektoren (6a, 6b, 6c) erstellt und ausgegeben und gegebenenfalls Zusatzinformationen (5) hinsichtlich dieser Gruppen ausgegeben werden,
und/oder
- **dass** auf die Suchanfrage eines Benutzers in der Datenbank (2) benachbarte Gruppen von Teilbildern (3a, 3b, 3c) und/oder Text-Informationen und/oder numerischen Informationen und/oder semantischen Informationen mit ähnlichen Feature-Vektoren (6a, 6b, 6c) ermittelt und ausgegeben werden und gegebenenfalls Zusatzinformationen (5) hinsichtlich dieser Gruppen ausgegeben werden.

10. Verfahren nach einem der Verfahren nach einem der Ansprüche 3 bis 9, **dadurch gekennzeichnet,**
- **dass** die als Ergebnis einer ersten Suchanfrage an die Datenbank (2) ermittelten Suchergebnisse, insbesondere Teilbilder (3a, 3b, 3c), Gruppeninformationen, Textinformationen, numerische Informationen und/oder semantische Informationen, zur Erstellung zumindest einer weiteren Suchanfrage verwendet werden, und
- **dass** die weitere Suchanfrage an zumindest eine weitere Datenbank (2a) übermittelt wird, und/oder
- **dass** zur Erstellung der zumindest einen weiteren Suchanfrage an die zumindest eine weitere Datenbank (2a) weitere als Ergebnis der ersten Suchanfrage an die Datenbank (2) ausgegebene Informationen verwendet werden,
- insbesondere, dass ausgegebene statistische Ergebnisse und/oder Ausgangsbilder (4a, 4b, 4c) und/oder ähnliche Teilbilder (3a, 3b, 3c) verwendet werden, wobei die Dimension der ausgegebenen Ausgangsbilder (4a, 4b, 4c) und/oder der ausgegebenen Teilbilder (3a, 3b, 3c) für die weitere Suchanfrage in der zumindest einen weiteren Datenbank (2a) gegebenenfalls reduziert wird.

11. Verfahren nach einem der vorangehenden Ansprüche, **dadurch gekennzeichnet, dass** einzelne Datensätze (1a, 1b, 1c) der Datenbank (2) ausschließlich für die Bildung einer Projektionsfunktion herangezogen werden, dem Benutzer jedoch nicht als Resultate von Abfragen zur Ansicht zur Verfügung gestellt werden.

12. Verfahren nach einem der vorangehenden Ansprüche, **dadurch gekennzeichnet, dass** in den Datensätzen (1a, 1b, 1c) Größenangaben betreffend die Pixelabmessungen oder Voxelabmessungen der Ausgangsbilder (4a, 4b, 4c) oder Teilbilder (3a, 3b, 3c) hinterlegt werden, oder
dass für die Ausgangsbilder (4a, 4b, 4c) oder Teilbilder (3a, 3b, 3c) die Pixelabmessungen oder Voxelabmessungen vorgegeben werden, indem nach ähnlichen, insbesondere vom selben Körperteil herrührenden, Referenz-Ausgangs- oder Referenz-Teilbildern mit bekannten Pixel- oder Voxelabmessungen gesucht wird
- anschließend durch Bildvergleich eine Skalierung gesucht wird, mit der das Ausgangs- oder Teilbild mit dem Referenz-Ausgangs- oder Referenz-Teilbild optimal in Übereinstimmung gebracht werden kann und
- ausgehend von dieser Skalierung und den bekannten Pixelabmessungen oder Voxelabmessungen des Referenz-Ausgangs- oder Referenz-Teilbilds die Pixelabmessungen oder Voxelabmessungen des Ausgangsbilds (4a, 4b, 4c) oder Teilbilds (3a, 3b, 3c) ermittelt und in der Datenbank (2) abgespeichert werden.

13. System zum Erstellen einer medizinischen Bilddatenbank, umfassend eine Trainingseinheit und eine der Trainingseinheit nachgeschaltete Indizierungseinheit, wobei die Trainingseinheit dazu ausgebildet ist,
unter Vorgabe von Datensätzen (1a, 1b, 1c), die Teilbilder (3a, 3b, 3c) von zwei- oder mehrdimensionalen Ausgangsbildern (4a, 4b, 4c) von Teilen des menschlichen Körpers umfassen wobei die jeweilige Position der Teilbilder (3a, 3b, 3c) eines Ausgangsbilds (4a, 4b, 4c) bezogen auf den menschlichen Körper ermittelt wird, wobei die Informationen zur Position der Teilbilder (3a, 3b, 3c) von einem neuronalen Netzwerk dazu genutzt werden, eine Projektion zur Schätzung der Positionen von Teilbildern (3a, 3b, 3c) zu erlernen, wobei
- für jedes Teilbild (3a, 3b, 3c) eines Ausgangsbilds (4a, 4b, 4c) die jeweilige Position in einem anatomischen Referenzatlas bekannt ist, und
- die einzelnen Ausgangsbilder (4a, 4b, 4c) oder Teilbilder (3a, 3b, 3c) gegebenenfalls mit textuellen, numerischen oder semantischen Zusatzinformationen (5a, 5b, 5c) versehen sind, und
- ein Teilbild (3a, 3b, 3c) auch einem gesamten Ausgangsbild (4a, 4b, 4c) entsprechen kann,
dadurch gekennzeichent, dass
- eine Projektion zur Gewinnung von Feature-Vektoren (6a, 6b, 6c) aus den Teilbildern (3a, 3b, 3c) zu erstellen,
- die, insbesondere visuell oder semantisch, ähnliche Teilbilder (3a, 3b, 3c) auf ähnliche Feature-Vektoren (6a, 6b, 6c) und/oder Feature-Vektoren (6a, 6b, 6c) mit geringem Abstand abbildet, und
- zur Vorbereitung der Ausführung der Projektion ein neuronales Netzwerk, insbesondere ein Convolutional-Neural-Network, auf der Basis vorgegebener Lern-Teilbilder zu erstellen, wobei die Datensätze (1a, 1b, 1c) oder ein Teil der Datensätze (1a, 1b, 1c) im Rahmen eines Metric-Learning-Verfahrens vom neuronalen Netzwerk dazu genutzt werden, die Projektion und das Erstellen der Feature-Vektoren (6a, 6b, 6c) einer komprimierten Repräsentation der in einem Lern-Teilbild enthaltenen Informationen aus Lern-Teilbildern sowie einer zu erreichenden vorgegebenen Ähnlichkeit zwischen den Lern-Teilbildern zu erlernen, wobei dem betreffenden Metric-Learning-Verfahren eine oder mehrere der folgenden Vorgaben zugrunde gelegt werden:
- Vorgabe von n-Tupeln von Lern-Teilbildern als ähnlich, die zueinander geringfügig verschoben, rotiert, verzerrt oder gestreckt sind und ausgehend vom selben Ausgangsbild (4a, 4b, 4c) erstellt wurden und/oder
- Vorgabe von n-Tupeln von Lern-Teilbildern als ähnlich, die ausgehend vom selben Teilbereich (3a, 3b, 3c) des Ausgangsbilds (4a, 4b, 4c) erstellt werden, wobei zumindest eines der Lern-Teilbilder gegenüber dem Teilbereich des Ausgangsbilds (4a, 4b, 4c) derart modifiziert wird, dass die Lern-Teilbilder unterschiedliches Rauschen und/oder, unterschiedliche Bildintensität und/oder unterschiedlichen Kontrast aufweisen und/oder
- Vorgabe von n-Tupeln von aus demselben Ausgangsbild (4a, 4b, 4c) stammenden Teilbereichen als Lern-Teilbilder, wobei die zu erreichende Ähnlichkeit zwischen den jeweiligen Lern-Teilbildern Bildern des n-Tupels vom räumlichen Abstand der betreffenden Teilbereiche im Ausgangsbild (4a, 4b, 4c) abhängt, wobei insbesondere Lern-Teilbilder umso ähnlicher angesehen werden, je näher die betreffenden Teilbereiche im Ausgangsbild (4a, 4b, 4c) beieinander liegen, und/oder
- Vorgabe von Lern-Teilbildern oder Gruppen von Lern-Teilbildern vom selben Ausgangsbild (4a, 4b, 4c) oder von unterschiedlichen Ausgangsbildern (4a, 4b, 4c), die aufgrund von mit den jeweiligen Ausgangsbildern (4a, 4b, 4c) hinterlegten externen Merkmalen von den textuellen, numerischen oder semantischen Zusatzinformationen als ähnlich anzusehen sind,
- wobei die Projektion gelernt wird mit der Zielfunktion, dass durch Abbildung von Paaren oder Gruppen von Teilbildern (3a, 3b, 3c) die räumliche Konstellation der Paare/Gruppen vor und nach der Projektion ähnlich ist und/oder
- wobei die Projektion basierend auf einer bekannten Abbildung von Teilbildern auf Positionen gelernt wird
- wobei zusätzlich zu den Feature-Vektoren (6a, 6b, 6c) die gelernte oder bekannte Positionsinformation in der Datenbank abgespeichert wird,
wobei die Indizierungseinheit dazu ausgebildet ist,
- auf die Teilbilder (3a, 3b, 3c) der Datensätze (1a, 1b, 1c) und/oder auf eine Anzahl weiterer Teilbilder weiterer Datensätze die von der Trainingseinheit erstellte Projektion anzuwenden und dementsprechend für diese Teilbilder jeweils zumindest ein Feature-Vektor erhalten wird, und
- die derart erstellten Feature-Vektoren, verknüpft mit den Datensätzen (1a, 1b, 1c) oder weiteren Datensätzen in einer Indexdatenstruktur hinterlegt werden, wobei die jeweilige Position der Teilbilder (3a, 3b, 3c) eines Ausgangsbilds (4a, 4b, 4c) bezogen auf den menschlichen Körper in dem anatomischen Referenzatlas ermittelt wird,
- wobei bei Vorliegen einer Suchanfrage die gesuchte Position im Körper ermittelt wird und in der Datenbank (2) nach Feature-Vektoren (6a, 6b, 6c) von Teilbildern (3a, 3b, 3c) gesucht wird, für die dieselbe Position in deren Datensätzen (1a, 1b, 1c) hinterlegt ist oder deren Position einen vom Benutzer vorgegebenen Abstandsschwellenwert zur gesuchten Position nicht überschreitet.

14. System nach Anspruch 13 umfassend eine der Trainingseinheit und der Indizierungseinheit nachgeschaltete Sucheinheit,
wobei die Sucheinheit dazu ausgebildet ist,
- eine Suchanfrage unter Vorgabe von zumindest einem Abfragebild (3') zu erstellen, wobei das Abfragebild (3') aus zumindest einem zwei- oder mehrdimensionalen Untersuchungsbild (4') oder in einer Untersuchungsbildsequenz ausgewählt ist,
- für das Abfragebild (3') einen Feature-Vektor nach der von der Trainingseinheit erstellten Projektion zu ermitteln,
- in der Datenbank (2) nach Datensätzen (1a, 1b, 1c) mit Feature-Vektoren (6a, 6b, 6c) zu suchen, die unter Zugrundelegung einer vorgegebenen Metrik in der Nähe des Feature-Vektors (6') des Abfragebilds (3') liegen, und
als Ergebnis der Suchanfrage Datensätze (1a, 1b, 1c) auszugeben, deren Teilbilder (3a, 3b, 3c) ein ähnliches Aussehen wie der Auswahlbereich aufweisen oder die semantisch relevant sind.

## Claims

1. Method for creating a medical image database,
a) wherein data records (1a, 1b, 1c) are created that comprise component images (3a, 3b, 3c) of two- or multidimensional initial images (4a, 4b, 4c) of parts of the human body, and wherein
- for each component image (3a, 3b, 3c) of an initial image (4a, 4b, 4c), the respective position in an anatomical reference atlas is known, and
- the individual initial images (4a, 4b, 4c) or component images (3a, 3b, 3c) are optionally provided with additional textual, numerical or semantic information (5a, 5b, 5c), and
- a component image (3a, 3b, 3c) can also correspond to an entire initial image (4a, 4b, 4c),
wherein
b) a projection to obtain feature vectors (6a, 6b, 6c) is created from the component images (3a, 3b, 3c),
- which, in particular visually or semantically, maps similar component images (3a, 3b, 3c) onto feature vectors (6a, 6b, 6c) at a small distance, and
- wherein, to prepare for the execution of the projection a neural network, in particular a convolutional neural network, is created on the basis of prescribed learning component images, wherein the data records (1a, 1b, 1c) or some of the data records (1a, 1b, 1c) are used by the neural network as part of a metric learning method to learn the projection and the creation of the feature vectors (6a, 6b, 6c) of a compressed representation of the information contained in a learning component image from learning component images or groups of learning component images as well as a prescribed similarity between the learning component images to be achieved, wherein the relevant metric learning method is based on one or more of the following prescriptions:
- prescribing as similar n-tuples of learning component images or groups of learning component images that are slightly shifted, rotated, distorted or elongated in relation to one another and have been created from the same initial image (4a, 4b, 4c), and/or
- prescribing as similar n-tuples of learning component images or groups of learning component images that are created starting from the same component region (3a, 3b, 3c) of the initial image (4a, 4b, 4c), wherein at least one of the learning component images is modified relative to the component region of the initial image (4a, 4b, 4c) in such a way that the learning component images have different noises and/or different image intensities and/or different contrasts, and/or
- prescribing as learning component images n-tuples of component regions originating from the same initial image or groups of initial images (4a, 4b, 4c), wherein the similarity between the respective learning component images of the n-tuple to be achieved depends on the spatial distance of the respective component regions in the initial image (4a, 4b, 4c), wherein in particular learning component images are viewed as being all the more similar, the closer the respective component regions in the initial image (4a, 4b, 4c) are to each other, and/or
- prescribing learning component images or groups of learning component images from the same initial image (4a, 4b, 4c) or from different initial images (4a, 4b, 4c), which are to be viewed as similar owing to external features of the additional textual, numerical or semantic information stored with the respective initial images (4a, 4b, 4c),
c) wherein the projection is applied to the component images (3a, 3b, 3c) of the data records (1a, 1b, 1c) and/or to a number of further component images of further data records, and accordingly at least one feature vector is obtained for each of these component images, and
d) wherein the feature vectors created in this way, linked to the initial images and/or data records (1a, 1b, 1c) or further data records, are stored in an index data structure,
wherein the respective position of the component images (3a, 3b, 3c) of an initial image (4a, 4b, 4c) relative to the human body is determined in the anatomical reference atlas,
and wherein the information on the position of the component images (3a, 3b, 3c) is used by a neural network to learn a projection for estimating the positions of component images (3a, 3b, 3c),
- wherein the projection is learned with the target function that, by mapping pairs or groups of component images (3a, 3b, 3c), the spatial configuration of the pairs/groups before and after the projection is similar and
- wherein the projection is learned based on a known mapping of component images onto positions,
- wherein, in addition to the feature vectors (6a, 6b, 6c), the learned or known position information is stored in the database,
- wherein, if a search request is made, the position in the body being searched is determined and the database (2) is searched for feature vectors (6a, 6b, 6c) of component images (3a, 3b, 3c) for which the same position is stored in their data records (1a, 1b, 1c) or whose position does not exceed a distance threshold prescribed by the user from the position searched for.

2. Method according to claim 1, **characterized in that** additional information (5) is stored in the data records (1a, 1b, 1c), wherein the additional information (5) is provided as text information and/or semantic information and/or numerical information, and
**in that** if text information or numerical information is available, the text information or numerical information is optionally stored in the database (2) as tags and/or semantic representations (7a, 7b, 7c), and
**in that** the additional information (5) is optionally used by the neural network to create the projection, wherein the projection is created in such a way that learning component images are prescribed as similar if they originate from initial images (4a, 4b, 4c) or correspond to component images (3a, 3b, 3c) to which the same additional information (5) is assigned.

3. Method according to one of claims 1 to 2, **characterized in that**,
- text information, numerical information and/or semantic information is provided by the user to create a search request,
- the text information or numerical information is optionally converted into tags and/or semantic representations (7a, 7b, 7c)
- if a search request is made, the database (2) is searched for feature vectors (6a, 6b, 6c) that have similar tags and/or semantic representations (7a, 7b, 7c).

4. Method according to one of claims 1 to 2, **characterized in that**,
- at least one query image (3'), in particular of a region of interest, is selected from at least one two- or multi-dimensional examination image (4') or in an examination image sequence to create a search request,
- for the query image (3') according to step c of claim 1, a feature vector is determined according to the learned projection,
- the database (2) is searched for data records (1a, 1b, 1c) with feature vectors (6a, 6b, 6c) which are based on a prescribed metric close to the feature vector (6') of the query image (3'), and
as a result of the search request, data records (1a, 1b, 1c) are output whose component images (3a, 3b, 3c) have a similar appearance to the selection region or which are semantically relevant, and
optionally, wherein, in order to search for similar images for an examination image (4') or an examination image sequence, a dimensionally reduced query component image which possibly has its dimensions reduced is created by creating a section, and this query component image is used for the search request.

5. Method according to one of claims 3 or 4, **characterized in that**, when searching the database (2) for similar feature vectors (6a, 6b, 6c), search results are excluded on the basis of the tags and/or semantic representations (7a, 7b, 7c) stored in the associated data records (1a, 1b, 1c) in the database (2),
in particular, **in that** search results are excluded under criteria prescribed by the user for the tags and/or semantic representations (7a, 7b, 7c).

6. Method according to one of claims 3 to 5, **characterized in that**,
as a result of the search request, a ranking of the data records (1a, 1b, 1c) determined in each case is created and output according to their similarity or correspondence to the tags and/or semantic representations (7a, 7b, 7c) of the search request and/or according to their similarity, determined by the difference from the respective feature vectors (6a, 6b, 6c), to at least one query image (3') of the search request.

7. Method according to one of claims 3 to 6, **characterized in that**,
- as a result of the search request, the text information and numerical information stored as tags in the respective data records (1a, 1b, 1c) and/or the semantic information stored as semantic representations (7a, 7b, 7c) are output and/or
- the tags of the component images (3a, 3b, 3c) similar to the selection region are statistically evaluated and the statistical result is output.

8. Method according to one of claims 3 to 7, **characterized in that** the examination images (4') and/or examination image sequences and/or text information and/or numerical information and/or semantic information underlying the search request are anonymized by the user before the search request is sent to the database (2).

9. Method according to one of claims 3 to 8, **characterized in that**,
- without a search request, groups of component images (3a, 3b, 3c) and/or text information and/or numerical information and/or semantic information with similar feature vectors (6a, 6b, 6c) are created and output directly in the database (2) on the basis of the data provided in the database and, if necessary, additional information (5) is output with regard to these groups,
and/or
- adjacent groups of component images (3a, 3b, 3c) and/or text information and/or numerical information and/or semantic information with similar feature vectors (6a, 6b, 6c) are determined and output following the search request by a user in the database (2) and, if necessary, additional information (5) with regard to these groups is output.

10. Method according to one of the methods according to one of claims 3 to 9, **characterized in that**
- the search results obtained as a result of an initial search request to the database (2), in particular component images (3a, 3b, 3c), group information, text information, numerical information and/or semantic information, are used to create at least one further search request, and
- the further search request is sent to at least one further database (2a), and/or
- further information that is output as a result of the initial search request to the database (2) is used to create the at least one further search request to the at least one further database (2a),
- in particular, statistical results and/or initial images (4a, 4b, 4c) and/or similar component images (3a, 3b, 3c) that are output are used, wherein the dimension of the output initial images (4a, 4b, 4c) and/or the output component images (3a, 3b, 3c) is reduced if necessary for the further search request in the at least one further database (2a).

11. Method according to one of the preceding claims, **characterized in that** individual data records (1a, 1b, 1c) of the database (2) are used exclusively for the formation of a projection function, but are not made available to the user for viewing as results of queries.

12. Method according to one of the preceding claims, **characterized in that** size specifications concerning the pixel dimensions or voxel dimensions of the initial images (4a, 4b, 4c) or component images (3a, 3b, 3c) are stored in the data records (1a, 1b, 1c), or
**in that**, for the initial images (4a, 4b, 4c) or component images (3a, 3b, 3c), the pixel dimensions or voxel dimensions are prescribed by searching for similar reference initial or reference component images, in particular from the same body part, with known pixel or voxel dimensions
- then, by comparing images, a scaling is sought that can optimally match the initial or component image with the reference initial or reference component image, and
- based on this scaling and the known pixel dimensions or voxel dimensions of the reference initial or reference component image, the pixel dimensions or voxel dimensions of the initial image (4a, 4b, 4c) or component image (3a, 3b, 3c) are determined and stored in the database (2).

13. System for creating a medical image database, comprising a training unit and an indexing unit downstream of the training unit,
wherein the training unit is designed,
prescribing data records (1a, 1b, 1c) that comprise component images (3a, 3b, 3c) of two- or multi-dimensional initial images (4a, 4b, 4c) of parts of the human body, wherein the respective position of the component images (3a, 3b, 3c) of an initial image (4a, 4b, 4c) is determined with respect to the human body,
wherein the information on the position of the component images (3a, 3b, 3c) is used by a neural network to learn a projection for estimating the positions of component images (3a, 3b, 3c), wherein
- for each component image (3a, 3b, 3c) of an initial image (4a, 4b, 4c), the respective position in an anatomical reference atlas is known, and
- the individual initial images (4a, 4b, 4c) or component images (3a, 3b, 3c) are optionally provided with additional textual, numerical or semantic information (5a, 5b, 5c), and
- a component image (3a, 3b, 3c) can also correspond to an entire initial image (4a, 4b, 4c),
**characterized in that**
- a projection to obtain feature vectors (6a, 6b, 6c) is created from the component images (3a, 3b, 3c),
- which, in particular visually or semantically, maps similar component images (3a, 3b, 3c) onto similar feature vectors (6a, 6b, 6c) and/or feature vectors (6a, 6b, 6c) at a small distance, and
- to prepare for the execution of the projection, a neural network, in particular a convolutional neural network, is created on the basis of prescribed learning component images, wherein the data records (1a, 1b, 1c) or some of the data records (1a, 1b, 1c) are used by the neural network as part of a metric learning method to learn the projection and the creation of the feature vectors (6a, 6b, 6c) of a compressed representation of the information contained in a learning component image from learning component images as well as a prescribed similarity between the learning component images to be achieved, wherein the relevant metric learning method is based on one or more of the following prescriptions:
- prescribing as similar n-tuples of learning component images that are slightly shifted, rotated, distorted or elongated in relation to one another and have been created from the same initial image (4a, 4b, 4c), and/or
- prescribing as similar n-tuples of learning component images that are created starting from the same component region (3a, 3b, 3c) of the initial image (4a, 4b, 4c), wherein at least one of the learning component images is modified relative to the component region of the initial image (4a, 4b, 4c) in such a way that the learning component images have different noises and/or different image intensities and/or different contrasts, and/or
- prescribing as learning component images n-tuples of component regions originating from the same initial image (4a, 4b, 4c), wherein the similarity between the respective learning component images of the n-tuple to be achieved depends on the spatial distance of the respective component regions in the initial image (4a, 4b, 4c), wherein in particular learning component images are viewed as being all the more similar, the closer the respective component regions in the initial image (4a, 4b, 4c) are to each other, and/or
- prescribing learning component images or groups of learning component images from the same initial image (4a, 4b, 4c) or from different initial images (4a, 4b, 4c), which are to be viewed as similar owing to external features of the additional textual, numerical or semantic information stored with the respective initial images (4a, 4b, 4c),
- wherein the projection is learned with the target function that, by mapping pairs or groups of component images (3a, 3b, 3c), the spatial configuration of the pairs/groups before and after the projection is similar and/or
- wherein the projection is learned based on a known mapping of component images onto positions,
- wherein, in addition to the feature vectors (6a, 6b, 6c), the learned or known position information is stored in the database,
wherein the indexing unit is designed
- to apply the projection created by the training unit to the component images (3a, 3b, 3c) of the data records (1a, 1b, 1c) and/or to a number of further component images of further data records, and accordingly at least one feature vector is obtained for each of these component images, and
- the feature vectors created in this way, linked to the data records (1a, 1b, 1c) or further data records, are stored in an index data structure, wherein the respective position of the component images (3a, 3b, 3c) of an initial image (4a, 4b, 4c) relative to the human body is determined in the anatomical reference atlas,
- wherein, if a search request is made, the position in the body being searched is determined and the database (2) is searched for feature vectors (6a, 6b, 6c) of component images (3a, 3b, 3c) for which the same position is stored in their data records (1a, 1b, 1c) or whose position does not exceed a distance threshold prescribed by the user from the position searched for.

14. System according to claim 13 comprising one of the training unit and search unit downstream of the indexing unit,
wherein the search unit is designed
- to create a search request prescribing at least one query image (3'), wherein the query image (3') is selected from at least one two- or multi-dimensional examination image (4') or in an examination image sequence,
- to determine a feature vector according to the projection created by the training unit for the query image (3'),
- to search the database (2) for data records (1a, 1b, 1c) with feature vectors (6a, 6b, 6c) which are based on a prescribed metric close to the feature vector (6') of the query image (3'), and
as a result of the search request, to output data records (1a, 1b, 1c) whose component images (3a, 3b, 3c) have a similar appearance to the selection region or which are semantically relevant.

## Revendications

1. Procédé de création d'une base de données d'images médicales,
a) dans lequel sont créés des ensembles de données (1a, 1b, 1c) qui comprennent des images partielles (3a, 3b, 3c) d'images initiales (4a, 4b, 4c) bidimensionnelles ou multidimensionnelles de parties du corps humain, étant entendu de préférence que :
- pour chaque image partielle (3a, 3b, 3c) d'une image initiale (4a, 4b, 4c), la position respective dans un atlas anatomique de référence est connue, et
- les images initiales (4a, 4b, 4c) ou images partielles (3a, 3b, 3c) individuelles sont éventuellement pourvues d'informations supplémentaires textuelles, numériques ou sémantiques (5a, 5b, 5c), et
- une image partielle (3a, 3b, 3c) peut également correspondre à une image initiale (4a, 4b, 4c) entière,
étant entendu que
b) une projection est créée pour obtenir des vecteurs caractéristiques (6a, 6b, 6c) à partir des images partielles (3a, 3b, 3c),
- laquelle met en correspondance, notamment visuellement ou sémantiquement, des images partielles (3a, 3b, 3c) similaires avec des vecteurs caractéristiques (6a, 6b, 6c) présentant une faible distance, et
- en préparation de l'exécution de cette projection un réseau neuronal, notamment convolutif, est créé à partir d'images partielles d'apprentissage prédéfinies, lesdits ensembles de données (1a, 1b, 1c) ou une partie de ces ensembles (1a, 1b, 1c) étant utilisés par le réseau neuronal, dans le cadre d'une méthode d'apprentissage métrique, pour apprendre la projection et la création des vecteurs caractéristiques (6a, 6b, 6c) d'une représentation compressée des informations contenues dans une image partielle d'apprentissage à partir d'images partielles d'apprentissage ou de groupes d'images partielles d'apprentissage ainsi que d'une similarité prédéfinie à atteindre entre les images partielles d'apprentissage, ladite méthode d'apprentissage métrique reposant sur une ou plusieurs des exigences suivantes :
- des n-uplets d'images partielles d'apprentissage ou de groupes d'images partielles d'apprentissage qui soient similaires, étant légèrement décalés, tournés, déformés ou étirés les uns par rapport aux autres et ayant été créés à partir de la même image initiale (4a, 4b, 4c), et/ou
- des n-uplets d'images partielles d'apprentissage ou de groupes d'images partielles d'apprentissage qui soient similaires, ayant été créés à partir de la même zone partielle (3a, 3b, 3c) de l'image initiale (4a, 4b, 4c), au moins une des images partielles d'apprentissage étant modifiée par rapport à la zone partielle de l'image initiale (4a, 4b, 4c) de telle façon que les images partielles d'apprentissage présentent un bruit différent et/ou une intensité d'image différente et/ou un contraste différent, et/ou
- des n-uplets de zones partielles provenant de la même image initiale ou de mêmes groupes d'images initiales (4a, 4b, 4c) qui servent d'images partielles d'apprentissage, la similarité à atteindre entre les images partielles d'apprentissage respectives images du n-uplet dépendant de la distance spatiale des zones partielles concernées dans l'image initiale (4a, 4b, 4c), les images partielles d'apprentissage étant notamment considérées comme d'autant plus similaires que les zones partielles concernées dans l'image initiale (4a, 4b, 4c) sont proches les unes des autres, et/ou
- des images partielles d'apprentissage ou des groupes d'images partielles d'apprentissage qui proviennent de la même image initiale (4a, 4b, 4c) ou d'images initiales (4a, 4b, 4c) différentes et devant être considérées comme similaires en raison de caractéristiques externes des informations supplémentaires textuelles, numériques ou sémantiques consignées avec les images initiales (4a, 4b, 4c) respectives,
c) dans lequel la projection est appliquée aux images partielles (3a, 3b, 3c) des ensembles de données (1a, 1b, 1c) et/ou à un certain nombre d'autres images partielles d'autres ensembles de données, moyennant quoi il est obtenu au moins un vecteur caractéristique pour chacune de ces images partielles, et
d) dans lequel les vecteurs caractéristiques ainsi créés sont consignés dans une structure de données d'indice en étant liés aux images initiales et/ou aux ensembles de données (1a, 1b, 1c) ou autres ensembles de données,
ladite position respective des images partielles (3a, 3b, 3c) d'une image initiale (4a, 4b, 4c) étant déterminée par rapport au corps humain dans l'atlas de référence anatomique, et les informations sur la position des images partielles (3a, 3b, 3c) étant utilisées par un réseau neuronal pour apprendre une projection permettant d'estimer les positions d'images partielles (3a, 3b, 3c),
- la projection étant apprise avec la fonction objectif selon laquelle une mise en correspondance de paires ou de groupes d'images partielles (3a, 3b, 3c) permet à la constellation spatiale des paires ou groupes d'être similaire avant et après la projection, et
- la projection étant apprise compte tenu d'une mise en correspondance connue d'images partielles et de positions,
- les informations de position apprises ou connues étant enregistrées, en sus des vecteurs caractéristiques (6a, 6b, 6c), dans la base de données,
- en présence d'une requête de recherche, la position recherchée étant déterminée dans le corps et une recherche étant effectuée dans la base de données (2) concernant des vecteurs caractéristiques (6a, 6b, 6c) d'images partielles (3a, 3b, 3c) pour lesquelles la même position est consignée dans leurs ensembles de données (1a, 1b, 1c) ou dont la position ne dépasse pas une valeur seuil de distance, prédéfinie par l'utilisateur, par rapport à la position recherchée.

2. Procédé selon la revendication 1, **caractérisé en ce que** des informations supplémentaires (5) sont consignées dans les ensembles de données (1a, 1b, 1c), lesdites informations supplémentaires (5) étant prédéfinies sous la forme d'informations textuelles et/ou sémantiques et/ou numériques, et
**en ce que**, éventuellement, en présence d'informations textuelles ou numériques, ces informations textuelles ou numériques sont consignées sous forme de balises et/ou de représentations sémantiques (7a, 7b, 7c) dans la base de données (2), et
**en ce que**, éventuellement, les informations supplémentaires (5) sont utilisées par le réseau neuronal pour créer la projection, ladite projection étant créée de telle manière que des images partielles d'apprentissage sont prédéfinies comme similaires lorsqu'elles proviennent d'images initiales (4a, 4b, 4c) ou correspondent à des images partielles (3a, 3b, 3c) auxquelles les mêmes informations supplémentaires (5) sont attribuées.

3. Procédé selon l'une des revendications 1 et 2, **caractérisé en ce que**
- pour créer une requête de recherche, des informations textuelles, des informations numériques et/ou des informations sémantiques sont indiquées par l'utilisateur,
- éventuellement les informations textuelles ou numériques sont converties en balises et/ou en représentations sémantiques (7a, 7b, 7c),
- en présence d'une requête de recherche, une recherche est effectuée dans la base de données (2) concernant des vecteurs caractéristiques (6a, 6b, 6c) possédant des balises et/ou des représentations sémantiques (7a, 7b, 7c) similaires.

4. Procédé selon l'une des revendications 1 et 2, **caractérisé en ce que**
- pour créer une requête de recherche, au moins une image de requête (3'), notamment d'une zone d'intérêt, est sélectionnée parmi moins une image d'examen (4') bidimensionnelle ou multidimensionnelle ou dans une séquence d'images d'examen,
- pour l'image de requête (3') selon l'étape c de la revendication 1, un vecteur caractéristique est déterminé selon la projection apprise,
- une recherche est effectuée dans la base de données (2) concernant des ensembles de données (1a, 1b, 1c) présentant des vecteurs caractéristiques (6a, 6b, 6c) qui, étant donné une métrique prédéterminée, sont proches du vecteur caractéristique (6') de l'image de requête (3'), et
en résultat de la requête de recherche, il est produit des ensembles de données (1a, 1b, 1c) dont les images partielles (3a, 3b, 3c) présentent un aspect similaire à la zone de sélection ou qui sont sémantiquement pertinents, et
dans lequel procédé, éventuellement, pour la recherche d'images similaires relativement à une image d'examen (4') ou à une séquence d'images d'examen, une image partielle de requête de moindre dimension et, le cas échéant, de dimensions réduites, est créée par création d'une coupure, et cette image partielle de requête est utilisée pour la requête de recherche.

5. Procédé selon l'une des revendications 3 et 4, **caractérisé en ce que**, lors de la recherche de vecteurs caractéristiques (6a, 6b, 6c) similaires dans la base de données (2), des résultats de recherche sont exclus en raison des balises et/ou des représentations sémantiques (7a, 7b, 7c) consignées dans les ensembles de données (1a, 1b, 1c) de la base de données (2) associés,
et notamment **en ce que** des résultats de recherche sont exclus selon des critères prédéfinis par l'utilisateur relativement aux balises et/ou aux représentations sémantiques (7a, 7b, 7c).

6. Procédé selon l'une des revendications 3 à 5, **caractérisé en ce que**, en résultat de la requête de recherche, un classement des ensembles de données (1a, 1b, 1c) respectivement déterminés est créé et délivré en fonction de leur similarité ou de leur correspondance avec les balises et/ou les représentations sémantiques (7a, 7b, 7c) de la requête de recherche, et/ou en fonction de leur similarité, déterminée par la distance des vecteurs caractéristiques (6a, 6b, 6c) concernés, avec au moins une image de requête (3') de la requête de recherche.

7. Procédé selon l'une des revendications 3 à 6, **caractérisé en ce que**
- en résultat de la requête de recherche, les informations textuelles ou numériques consignées sous la forme de balises dans les ensembles de données respectifs (1a, 1b, 1c) et/ou les informations sémantiques consignées sous la forme de représentations sémantiques (7a, 7b, 7c) sont délivrées, et/ou
- les balises des images partielles (3a, 3b, 3c) similaires à la zone de sélection sont évaluées statistiquement et le résultat statistique est délivré.

8. Procédé selon l'une des revendications 3 à 7, **caractérisé en ce que** les images d'examen (4') et/ou les séquences d'images d'examen et/ou les informations textuelles et/ou les informations numériques et/ou les informations sémantiques sur lesquelles repose la demande de recherche sont anonymisées avant que l'utilisateur ne transmette la demande de recherche à la base de données (2).

9. Procédé selon l'une des revendications 3 à 8, **caractérisé en ce que**
- sans requête de recherche, des groupes d'images partielles (3a, 3b, 3c) et/ou des informations textuelles et/ou des informations numériques et/ou des informations sémantiques présentant des vecteurs caractéristiques (6a, 6b, 6c) similaires sont créés et délivrés directement compte tenu des données présentes dans la base de données (2) et, le cas échéant, des informations supplémentaires (5) sont délivrées concernant ces groupes,
et/ou
- en réaction à une requête de recherche d'un utilisateur dans la base de données (2), des groupes voisins d'images partielles (3a, 3b, 3c) et/ou des informations textuelles et/ou des informations numériques et/ou des informations sémantiques présentant des vecteurs caractéristiques (6a, 6b, 6c) similaires sont déterminés et délivrés et, le cas échéant, des informations supplémentaires (5) sont délivrées concernant ces groupes.

10. Procédé selon l'une des revendications 3 à 9, **caractérisé en ce que**
- les résultats de recherche déterminés en résultat d'une première requête de recherche dans la base de données (2), notamment des images partielles (3a, 3b, 3c), des informations de groupe, des informations textuelles, des informations numériques et/ou des informations sémantiques, sont utilisés pour créer au moins une autre requête de recherche, et
- l'autre requête de recherche est transmise à au moins une autre base de données (2a), et/ou
- pour créer l'au moins une autre requête de recherche dans l'au moins une autre base de données (2a), il est utilisé d'autres informations délivrées à la base de données (2) en résultat de la première requête de recherche,
- il est utilisé notamment des résultats statistiques délivrés et/ou des images initiales (4a, 4b, 4c) délivrées et/ou des images partielles (3a, 3b, 3c) similaires délivrées, la dimension des images initiales (4a, 4b, 4c) délivrées et/ou des images partielles initiales (3a, 3b, 3c) délivrées étant éventuellement réduite pour ladite autre requête de recherche dans l'au moins une autre base de données (2a).

11. Procédé selon l'une des revendications précédentes, **caractérisé en ce que** des ensembles de données (1a, 1b, 1c) individuels de la base de données (2) sont utilisés exclusivement pour la formation d'une fonction de projection et ne sont pas mis à la disposition de l'utilisateur pour visualisation sous la forme de résultats de requêtes.

12. Procédé selon l'une des revendications précédentes, **caractérisé en ce que** des indications de taille concernant les dimensions en pixels ou en voxels des images initiales (4a, 4b, 4c) ou des images partielles (3a, 3b, 3c) sont consignées dans les ensembles de données (1a, 1b, 1c), ou
**en ce que** les dimensions en pixels ou en voxels sont prédéfinies pour les images initiales (4a, 4b, 4c) ou les images partielles (3a, 3b, 3c) par recherche d'images initiales de référence ou d'images partielles de référence similaires avec des dimensions en pixels ou en voxels connues, en particulier provenant de la même partie du corps,
- ensuite, par comparaison des images, une mise à l'échelle est ensuite recherchée, grâce à laquelle l'image initiale ou partielle peut être alignée de manière optimale avec l'image initiale ou partielle de référence, et
- compte tenu de cette mise à l'échelle et des dimensions en pixels ou en voxels connues de l'image initiale de référence ou de l'image partielle de référence, les dimensions en pixels ou en voxels de l'image initiale (4a, 4b, 4c) ou de l'image partielle (3a, 3b, 3c) sont déterminées et enregistrées dans la base de données (2).

13. Système de création d'une base de données d'images médicales, comprenant une unité d'apprentissage et une unité d'indexation postérieure à l'unité d'apprentissage, ladite unité d'apprentissage étant conçue pour,
étant donné des ensembles de données (1a, 1b, 1c) comprenant des images partielles (3a, 3b, 3c) d'images initiales (4a, 4b, 4c) bidimensionnelles ou multidimensionnelles de parties du corps humain, ladite position respective des images partielles (3a, 3b, 3c) d'une image initiale (4a, 4b, 4c) étant déterminée par rapport au corps humain,
les informations sur la position des images partielles (3a, 3b, 3c) étant utilisées par un réseau neuronal pour apprendre une projection permettant d'estimer les positions d'images partielles (3a, 3b, 3c), étant entendu que :
- pour chaque image partielle (3a, 3b, 3c) d'une image initiale (4a, 4b, 4c), la position respective dans un atlas anatomique de référence est connue, et
- les images initiales (4a, 4b, 4c) ou images partielles (3a, 3b, 3c) individuelles sont éventuellement pourvues d'informations supplémentaires textuelles, numériques ou sémantiques (5a, 5b, 5c), et
- une image partielle (3a, 3b, 3c) peut également correspondre à une image initiale (4a, 4b, 4c) entière,
**caractérisé en ce que**
- créer une projection pour obtenir des vecteurs caractéristiques (6a, 6b, 6c) à partir des images partielles (3a, 3b, 3c),
- laquelle met en correspondance, notamment visuellement ou sémantiquement, des images partielles (3a, 3b, 3c) similaires avec des vecteurs caractéristiques (6a, 6b, 6c) similaires et/ou avec des vecteurs caractéristiques (6a, 6b, 6c) présentant une faible distance, et
- en préparation de l'exécution de cette projection, créer un réseau neuronal, notamment convolutif, à partir d'images partielles d'apprentissage prédéfinies, lesdits ensembles de données (1a, 1b, 1c) ou une partie de ces ensembles (1a, 1b, 1c) étant utilisés par le réseau neuronal, dans le cadre d'une méthode d'apprentissage métrique, pour apprendre la projection et la création des vecteurs caractéristiques (6a, 6b, 6c) d'une représentation compressée des informations contenues dans une image partielle d'apprentissage à partir d'images partielles d'apprentissage ainsi que d'une similarité prédéfinie à atteindre entre les images partielles d'apprentissage, ladite méthode d'apprentissage métrique reposant sur une ou plusieurs des exigences suivantes :
- des n-uplets d'images partielles d'apprentissage qui soient similaires, étant légèrement décalés, tournés, déformés ou étirés les uns par rapport aux autres et ayant été créés à partir de la même image initiale (4a, 4b, 4c), et/ou
- des n-uplets d'images partielles d'apprentissage qui soient similaires, ayant été créés à partir de la même zone partielle (3a, 3b, 3c) de l'image initiale (4a, 4b, 4c), au moins une des images partielles d'apprentissage étant modifiée par rapport à la zone partielle de l'image initiale (4a, 4b, 4c) de telle façon que les images partielles d'apprentissage présentent un bruit différent et/ou une intensité d'image différente et/ou un contraste différent, et/ou
- des n-uplets de zones partielles provenant de la même image initiale (4a, 4b, 4c) qui servent d'images partielles d'apprentissage, la similarité à atteindre entre les images partielles d'apprentissage respectives images du n-uplet dépendant de la distance spatiale des zones partielles concernées dans l'image initiale (4a, 4b, 4c), les images partielles d'apprentissage étant notamment considérées comme d'autant plus similaires que les zones partielles concernées dans l'image initiale (4a, 4b, 4c) sont proches les unes des autres, et/ou
- des images partielles d'apprentissage ou des groupes d'images partielles d'apprentissage qui proviennent de la même image initiale (4a, 4b, 4c) ou d'images initiales (4a, 4b, 4c) différentes et devant être considérées comme similaires en raison de caractéristiques externes des informations supplémentaires textuelles, numériques ou sémantiques consignées avec les images initiales (4a, 4b, 4c) respectives,
- la projection étant apprise avec la fonction objectif selon laquelle une mise en correspondance de paires ou de groupes d'images partielles (3a, 3b, 3c) permet à la constellation spatiale des paires ou groupes d'être similaire avant et après la projection, et/ou
- la projection étant apprise compte tenu d'une mise en correspondance connue d'images partielles et de positions,
- les informations de position apprises ou connues étant enregistrées, en sus des vecteurs caractéristiques (6a, 6b, 6c), dans la base de données ;
ladite unité d'indexation étant conçue pour
- appliquer la projection créée par l'unité d'apprentissage aux images partielles (3a, 3b, 3c) des ensembles de données (1a, 1b, 1c) et/ou à un certain nombre d'autres images partielles d'autres ensembles de données, moyennant quoi il est obtenu au moins un vecteur caractéristique pour chacune de ces images partielles, et
- consigner les vecteurs caractéristiques ainsi créés, liés aux ensembles de données (1a, 1b, 1c) ou à d'autres ensembles de données, dans une structure de données d'indice, ladite position respective des images partielles (3a, 3b, 3c) d'une image initiale (4a, 4b, 4c) étant déterminée par rapport au corps humain dans l'atlas de référence anatomique,
- en présence d'une requête de recherche, la position recherchée étant déterminée dans le corps et une recherche étant effectuée dans la base de données (2) concernant des vecteurs caractéristiques (6a, 6b, 6c) d'images partielles (3a, 3b, 3c) pour lesquelles la même position est consignée dans leurs ensembles de données (1a, 1b, 1c) ou dont la position ne dépasse pas une valeur seuil de distance, prédéfinie par l'utilisateur, par rapport à la position recherchée.

14. Système selon la revendication 13 comprenant une unité de recherche postérieure à l'unité d'apprentissage et à l'unité d'indexation,
ladite unité de recherche étant conçue pour
- créer une requête de recherche, étant donné au moins une image de requête (3'), l'image de requête (3') étant sélectionnée parmi au moins une image d'examen (4') bidimensionnelle ou multidimensionnelle ou dans une séquence d'images d'examen,
- déterminer, pour l'image de requête (3'), un vecteur caractéristique selon la projection créée par l'unité d'apprentissage,
- effectuer une recherche dans la base de données (2) concernant des ensembles de données (1a, 1b, 1c) présentant des vecteurs caractéristiques (6a, 6b, 6c) qui, étant donné une métrique prédéterminée, sont proches du vecteur caractéristique (6') de l'image de requête (3'), et
produire, en résultat de la requête de recherche, des ensembles de données (1a, 1b, 1c) dont les images partielles (3a, 3b, 3c) présentent un aspect similaire à la zone de sélection ou qui sont sémantiquement pertinents.
